# EUROPÄISCHE PATENTSCHRIFT

(11) **EP 0 794 731 B1**
(45) Veröffentlichungstag und Bekanntmachung des Hinweises auf die Patenterteilung: **24.04.2002**
(21) Anmeldenummer: 95936879.6
(22) Anmeldetag: 28.11.1995
(51) Int. Cl.: A61B 5/103

(54) **EINRICHTUNG UND VERFAHREN ZUR HAUTUNTERSUCHUNG**
DEVICE AND METHOD FOR SKIN EXAMINATION
DISPOSITIF ET PROCEDE POUR EXAMEN DE LA PEAU

(30) Priorität: 01.12.1994 AT 223394
(43) Veröffentlichungstag der Anmeldung: 17.09.1997
(73) Patentinhaber: Artner, Norbert, 1130 Wien (AT)
(72) Erfinder: Binder, Michael, A-1070 Wien (AT)
(74) Vertreter: Wildhack, Helmut, Dr. Dipl.-Ing.
(86) Internationale Anmeldenummer: AT9500231
(87) Internationale Veröffentlichungsnummer: WO9616698

(56) Entgegenhaltungen:
- EP-A- 0 475 803
- DE-U- 9 417 828
- FR-A- 2 658 410
- US-A- 4 398 541
- US-A- 4 930 872

## Beschreibung

Die optische Untersuchung der Haut, wobei dieselbe im gegebenenfalls schrägen Auflicht entweder mit freiem Auge, mittels Lupe oder Mikroskop oder mit einer photographischen Kamera beobachtet wird, ist eine bekannte und als solche gut ausgebaute Technik. So stellt die Epilumineszenzmikroskopie (ELM) von pigmentierten Läsionen der Haut eine heute etablierte Methode zur Früherkennung von Hautkrebs, sowie zur Differentialdiagnose von pigmentierten Hautläsionen dar.

Das dabei zur Anwendung kommende physikalisch-optische Prinzip ist einfach: Die Haut bzw. eine pigmentierte Hautläsion wird mit einer 4- bis 10-Fach-Vergrößerungseinrichtung, wie z.B. Lupeneinrichtung oder Operationsmikroskop betrachtet. Eine meist zumindest im wesentlichen orthogonal auf die Hautoberfläche gerichtete Lichtquelle beleuchtet das Beobachtungsfeld. Um den Effekt der ELM nützen zu können, wird zwischen Lupeneinrichtung und Hautoberfläche eine plane Glasfläche eingebracht. Zwischen Glasfläche und der Hautoberfläche werden einige Tropfen Immersionsfluid, meist -öl aufgetragen. Durch diese Technik wird der von der üblicherweise rauhen Hautoberfläche diffus reflektierte Lichtanteil vermindert, und es ist dem Betrachter möglich, bis an die Grenzzone zwischen Oberhaut und Unterhaut zu blicken. Gerade an dieser Grenzzone, der sogenannten dermoepidermalen Junktionszone, sowie in den benachbarten Zonen, wie in der Epidermis oder in oberflächlichen Schichten der Dermis, ist der pathologische Prozeß von pigmentierten Läsionen lokalisiert.

Der geschulte Dermatologe kann daher durch den Einsatz der ELM genaueren Einblick in die Anatomie pigmentierter Läsionen erhalten und somit weitaus früher bösartige - von gutartigen Läsionen unterscheiden.

Eine große Anzahl von intemationalen Publikationen bestätigt den benefiziellen Effekt der ELM zur Frühdiagnose des malignen Melanoms der Haut.

Die eben beschriebene traditionelle Auflichtmikroskopie weist, wie die Praxis zeigt, neben den o.a. durchaus positiven Wirkungen insbesondere folgende Nachteile auf:
- Bis jetzt wird ELM mit Handlupen bzw. Operationsmikroskopen durchgeführt. Eine Dokumentation sowie Diskussion der dadurch entstehenden Abbildungen ist somit nur erschwert und unter Einsatz einer schwerfälligen Archivierungs- und Dokumentations-Infrastruktur möglich.
- Durch das Aufbringen von Immersionsöl auf die Haut und unter die beschriebene Glasplatte treten üblicherweise Lufteinschlüsse auf, die die Diagnose erschweren.
- Bei der - verständlicherweise besonders - wichtigen Dokumentation von pigmentierten Läsionen wird bis heute ein unkonventionelles fotografisches Verfahren verwendet, das selbst eine Reihe von Nachteilen birgt:
   - Der fotografische Prozeß ist nicht sofort evaluierbar, zudem müssen immer Belichtungsreihen durchgeführt werden.
   - Der fotografische Prozeß ist nicht standardisiert; es seien hier z.B. die Variabilität der Filmcharge, die Variabilität des Naßentwicklungsprozesses, wodurch die erhaltenen Ergebnisse zumindest in der Graustufen- bzw. Farbbeurteilung nicht miteinander vergleichbar sind, erwähnt.
   - Der fotografische Prozeß ist nur über weitere Hilfseinrichtungen, wie z.B. Diaprojektor, für Vergleichsuntersuchungen wieder verfügbar.
   - Der Selektions- und Archivierungsaufwand erfordert relativ hohe Mühe und Kosten.

Zusammenfassend ist also festzustellen, daß zur Untersuchung und zur Begutachtung pigmentierter Hautläsionen derzeit ein zwar effizientes, aber von vielen Stör- und Kostenfaktoren begleitetes Verfahren angewandt wird.

Die Erfindung hat sich die Aufgabe gestellt, den heutigen Erfordernissen entsprechende Untersuchungs- und Bildaufnahme-Einrichtungen für Humanhaut zu schaffen und die Untersuchungsmethodik einschließlich Aufnahmetechnik selbst zu verbessern und so eine moderne Voraussetzungen nutzende Diagnose- und Dokumentationstechnik bereitzustellen. Dabei soll nicht nur die Effizienz der ELM bei Hautuntersuchungen allgemein und im speziellen in der Frühdiagnostik pigmentierter Hautläsionen signifikant steigen, sondern auch eine deutliche Verringerung des Manipulationsaufwandes und der Erhaltungskosten, insbesondere auch hinsichtlich des Diagnoseund Dokumentationssystems erzielt werden.

Um den Weg, der zur Erfindung führt, zu verdeutlichen, wird gleich hier unter Bezugnahme auf die erst später behandelte Zeichnung und deren genaue Erläuterung folgendes ausgeführt:

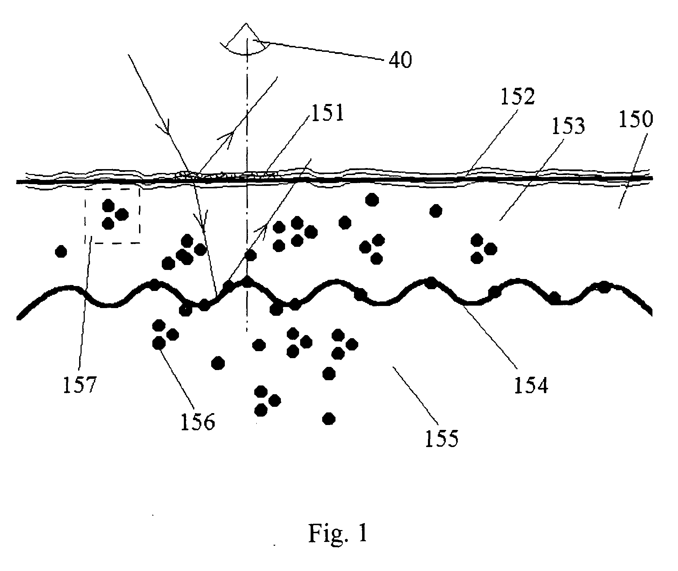

Innerhalb allgemeiner physikalischer Überlegungen zur Auflichtmikroskopie zeigt die Fig. 1 schematisch eine übliche Betrachtung der Haut ohne auflichtmikroskopische Techniken auf, wobei diese eine Übersicht eines stark vergrößerten Vertikalschnitts durch menschliche Haut darstellt. Die vollen Kreise stellen die Pigmentzellen dar. Die Anordnung dieser Pigmentzellen ist für den Krankheitsprozeß an pigmentierten Hautläsionen charakteristisch.

Drei wesentliche Grenzschichten sind hier von Bedeutung: a) Grenzschicht zwischen Luft und der Hornschicht, b) Grenzschicht zwischen Oberhaut-Epidermis und Unterhaut-Dermis, c) Grenzschicht zwischen den pigmentierten Zellen in der Haut und ihrem umgebenden Medium. Die Dicke der Oberhaut-Epidermis beträgt rund 0,1 - 0,3 mm.

Auf die Haut wird ein Lichtstrahl gerichtet. Der größte Anteil dieses Lichtstrahls wird bereits an der obersten Grenzschicht zwischen Luft und Homschicht - symbolisch als Lichtstrahl dargestellt - diffus reflektiert. Ein weitaus geringerer Anteil wird von den anderen, tiefer liegenden Grenzschichten reflektiert. Der größte Anteil des eintreffenden Lichtes wird in den oberen Anteilen der Unterhaut-Dermis absorbiert.

Die Fig. 1 zeigt deutlich, daß es ohne Anwendung auflichtmikroskopischer Techniken deshalb praktisch nicht möglich ist, in die Oberhaut-Epidermis einzusehen, da der größte Anteil des Lichtes reflektiert wird und an Quantität den eindringenden Lichtanteil, von dem auch wieder nur wenig reflektiert wird, überwiegt.

Wenn nun eine Betrachtung der Haut mittels konventioneller auflichtmikroskopischer Techniken vorgenommen werden soll, muß die Aufbringung einer planen Glasfläche, sowie eines Tropfens Immersionsöl zwischen der selben und der Hautoberfläche erfolgen, welches Öl den gleich an der obersten Grenzschicht diffus reflektierten Lichtanteil reduziert. Es kann nun relativ mehr Licht von den tieferliegenden Anteilen wahrgenommen werden. Der Nachteil dieser Methode liegt insbesondere in dem Benetzen der Hautoberfläche mit Öl selbst, sowie in der Bildung störender Luftblasen im Ölfilm, wobei neue und optisch störende Grenzschichten zwischen dünnen und dichten Medien entstehen.

Es wurde nun versucht, eine für den untersuchenden Arzt problemlos und einfach handzuhabende, den heutigen Erfordernissen entsprechende unf für eine moderne Diagnose- und Dokumentationstechnik besonders geeignete, den Patienten und dessen Befinden praktisch nicht beeinträchtigende, Einrichtung zur optischen Untersuchung von Humanhaut und insbesondere von Läsionen derselben bereitzustellen und im Zusammenhang damit das störende materielle Element der Beobachtung, nämlich das Immersionsöl und dessen Aufbringung auf die Haut unter der Transparentplatte zu vermeiden und auf prinzipiell andere Weise, am günstigsten durch Nutzung eines immateriellen, physikalischen Phänomens, das prinzipielle Ziel einer Eliminierung des störenden diffusen Hauptanteils des von der äußersten Hautfläche reflektierten Lichtes zu erreichen.

Dabei führten diverse Überlegungen und vergleichende Untersuchungsreihen zu dem Ergebnis, daß dieses Grundziel einschließlich weiteren Ausgestaltungen durch eine Umstellung der Haut-Untersuchungs- und -Diagnosetechnik auf den aktiven und/oder passiven Einsatz von polarisiertem Licht mit überraschenden Erfolgen auf diesem Gebiet erreicht werden kann.

Demgemäß ist Gegenstand der vorliegenden Erfindung eine neue Einrichtung zur optischen Untersuchung von Humanhaut gemäß Oberbegriff des Patentanspruches 1.

An dieser Stelle ist festzuhalten, daß Oberflächen-Untersuchungen und Analysen unter Einsatz von polarisiertem Licht und Polarisationsfiltern in größerer Zahl bekannt geworden sind. Was nun den Einsatz dieser Technik und dafür vorgeschlagene Geräte betrifft sei hier besonders auf die US-A 5 198 875 hingewiesen.

Nachteil dieses Gerätes ist es, daß eine bei jeder Untersuchung innerhalb einer zeitlich aufeinanderfolgende Sitzungen umfassenden Untersuchungsreihe tatsächlich jeweils reproduzierbare Abbildung einer Hautstelle bei Einsatz der bekannten hautseitig offenen Geräte nicht erreichbar ist, da es nicht möglich ist, eine von Untersuchungstermin zu Untersuchungstermin idente, dreidimensionale, reproduktive Topografie der Hautstelle zu erzielen, die allein vergleichende Untersuchungen überhaupt effektiv erscheinen läßt. Nur unter "Verebnung" der Haut sind reproduzier- und untereinander vergleichbare Abbildungen zu verschiedenen Zeitpunkten erzielbar. Eine Verebnung gelingt durch Anordnung einer Glasplatte, die, siehe oben, jedoch den Einsatz eines Immersionsöles nötig macht.

Im wesentlichen ähnlich sind die Nachteile des nur für eine Hautpflege-Kontrolle vorgesehenen Hautbetrachtungsgerätes gemäß DE 94 17 828 U1, welches eine handhabungsfreundliche Bauweise mit tubusartigem Gehäuseteil mit Lichteinlaßöffnung und fest zwischen derselben und einer Lichtquelle angeordnetem Polaroidfilter sowie einer CCD-Sensorkamera aufweist und einen sich im Winkel vom Gehäuseteil wegerstreckenden, hohlen Griffteil, in welchem ein Mechanismus zum Verschieben eines zweiten, ebenfalls in seiner Polarisation nicht veränderlichen Polaroidfilters untergebracht ist.

Erfindungsgemäß ist diese Einrichtung durch die im Kennzeichen des Patentanspruches 1 angeführten Merkmale gekennzeichnet. Ergänzend sei ausgeführt, daß als Vergrößerungseinrichtung, z.B. Lupe, Stereolupe, Mikroskop oder dessen Objektiv zu nennen sind, daß die Beleuchtungseinrichtung u.a. Lichtwegänderungs-Lichtbündelungs und/oder Lichtverteilungselemente umfassen kann und daß die beiden Polarisationseinrichtungen, also Polarisator und Analysator, unabhängig voneinander ver- und einstellbar sind.

Wenn nun im Sinne der Erfindung mit der neuen Untersuchungseinrichtung eine Betrachtung der Haut unter Anwendung eines Polarisationsfilters ohne die Verwendung von Immersionsöl erfolgt, ergibt sich folgende Situation: Licht strahlt auf die Hautoberfläche ein. Der an der ersten Grenzschicht reflektierte Anteil ist demnach linear polarisiert und kann bei geeignet ("senkrecht") eingestellter Polarisationsebene eines Polarisationsfilters bzw. Analysators - vor der Objektivebene einer Lupe oder eines Mikroskops liegend-, ausgefiltert werden. Es ist dadurch ermöglicht, ohne den Einsatz von Immersionsöl den auflichtmikroskopischen Effekt zu erreichen. Durch Drehen des Analysators ist es möglich, beide Arten der Darstellung, einerseits eine rein konventionelle Lupendarstellung zur Beurteilung der Oberfläche bei nichtgedrehtem Pol-Filter und andererseits die auflichtmikroskopische, Immersionsfluid benötigende Darstellung (ohne dasselbe), in einer Sitzung zu erzielen. Dieses Verfahren ermöglicht noch keine relative Verstärkung der Sichtbarkeit des Melaninpigmentes, da nur ein Polfilter verwendet wird. Für eine Routineapplikation hat sich dieses Prinzip der Anwendung des Analysators deswegen als günstig erwiesen, da relativ viel Licht für die Betrachtung einer Hautstelle, z.B. einer Pigmentläsion zur Verfügung steht.

Diese mit der neuen Einrichtung ermöglichte, in ihrem Informationsgehalt wesentlich verdichtete Methodik umfaßt eine Betrachtung der Haut unter Einsatz von polarisiertem Auflicht und Durchgang der reflektierten Anteile durch ein weiteres Pol-Filter (Analysator) ohne Verwendung von Immersionsöl. Linear polarisiertes Licht strahlt auf die Hautoberfläche ein. Der an der ersten Grenzschicht reflektierte Anteil ist nun auch linear polarisiert und kann durch entsprechende Drehung der Polarisationsebene des zweiten Polarisationsfilters (Analysator) vor der Objektivebene einer optischen Vergrößerungseinrichtung, ausgefiltert werden. Es ist dadurch möglich, ohne die Anwendung von Immersionsöl zumindest den vollen auflichtmikroskopischen Effekt zu erreichen. Durch Drehen des zweiten, im Strahlengang des reflektierten Lichtes liegenden Polarisationsfilters ist es zudem möglich, beide Arten der Darstellung, einerseits die konventionelle Lupendarstelltung zur Beurteilung der Oberfläche, andererseits die auflichtmikroskopische, sonst auf Immersionsfluid angewiesene, Darstellung in einer Sitzung zu erzielen. Da zudem das in den Pigmentzellen-Melanozyten enthaltene Pigment eine optisch aktive Substanz darstellt bzw. enthält, läßt sich durch geeignete Position des Analysators der optische Eindruck dieser für die Diagnose und Früherkennung wichtigen Zellpopulation noch verstärken.

Was die konkrete Bauweise des neuen Untersuchungsgerätes betrifft, haben sich zwei Konstruktionen als im Routine-Untersuchungs-Betrieb besonders günstig erwiesen, deren eine ihr Bild-Aufnahmegerät und auch die Beleuchtungseinrichtung in Nähe des Beobachtungsfeldes vorsieht, während die andere für eine die thermische Belastung der Patientenhaut minimierende räumliche Dislozierung der Beleuchtungseinrichtung und letztlich auch des Bild-Aufnahmegerätes von der Haut Sorge trägt.

Bevorzugterweise ist das Bildaufnahmegerät eine Klein- oder Kleinst-Videokamera.

Bevorzugte Polarisator- und Analysator- Einrichtungen sind geringsten Platzbedarf aufweisende moderne Polarisationsfolienfilter. Für Spezialzwecke kann von der tatsächlich planaren Ausbildung der gehäuse-abschließenden Transparentplatte abgegangen werden und eine leicht konvexe oder konkave Platte Einsatz finden.

Insbesondere aus Hygiene- und Reinigungsgründen ist als Transparentplatten-Material Glas und hiebei wieder besonders kratzfestes, robustes, gehärtetes Mineralglas bevorzugt.

Hohe Einsatzflexibilität, Manipulationssicherheit und Körpertopografie-Kompatibilität zeigt eine konkrete vorteilhafte Ausbildungsvariante des neuen Kompakt-Untersuchungsgerätes gemäß **Anspruch 2**. Sie kommt daneben auch noch der heute so notwendigen Tendenz zur Miniaturisierung in hohem Maße entgegen.

Es soll hier betont werden, daß moderne Flachbandtechnik bei den Leitungen besonders bevorzugt wird. Als Steuer-, Schalt- und Speichereinheit haben sich moderne (Personal-) Computer bewährt, zur Bildwiedergabe moderne Bildschirme hoher Auflösung; besonders bevorzugte und in der ärztlichen Praxis bewährte Regel-Schaltorgane sind insbesondere Trackballs und Mikroschalter bzw. - taster.

Besonders wichtig sind optimale Beleuchtungs- und damit Beobachtungsbedingungen, insbesondere auch im Hinblick auf Reproduzierbarkeit auch nach längeren Zeitintervallen. In diesem Sinne ist eine Ausgestaltung der neuen Einrichtung gemäß **Anspruch 3** besonders vorteilhaft. Die gleichmäßige Lichtstreuung kann bevorzugt durch Strahl-Rauhung des Lichtstreuprismas erzielt werden.

Die im **Anspruch 4** niedergelegte, bevorzugte Ausführungsform kann in hohem Maße eine freie Drehbarkeit des Analysators bei gleichzeitig hoher Stabilität des Gehäuses und damit hohe Beobachtunsgenauigkeit gewährleisten.

Eine insbesondere, für Reinigung und Desinfektion günstige, mehrteilige Ausbildungsform des Gehäuses bildet den Gegenstand des **Anspruches 5**.

Zur Unterbringung der wesentlichen Funktionselemente und -organe auf kleinstem Raum hat sich eine Bauform des neuartigen Haut-Untersuchungsgerätes mit abgesetzt gestaltetem Analysator-Polarisationsfilter als ausgesprochen günstig erwiesen, wie sie aus **Anspruch 6** hervorgeht.

Der o.a. zweiten prinzipiellen Bauform mit Funktionselementen und -organen im Griffteil entsprechend, ist eine Bauvariante des neuen Gerätes gemäß **Anspruch 7** gestaltet. Bezüglich Lichtausbeute ist als Lichtumlenk-Element ein Umlenkspiegel bevorzugt. Ein an Außenund Innenseite mit lichtreflektierendem Material beschichtetes Lichtleitrohr vermindert unerwünschte Lichtausbeuteverluste.

Eine insbesondere für raschen Lampenwechsel und Lampenersatz besonders geeignete Bauform der neuen Einrichtung weist die im **Anspruch 8** wiedergegebenen Hauptmerkmale auf. Kostengünstig und leicht verfügbare Lichtemissionselemente sind bevorzugterweise konventionell gebaute Glühlampen.

Die oben angeführte, eine minimale thermische Belastung der Haut sicherstellende Dislokation der Beleuchtungseinrichtung läßt sich durch eine bevorzugte Bauform gemäß **Anspruch 9** erreichen.

Auch die Ausführungsform der neuen Untersuchungseinrichtung gemäß **Anspruch 10** gewährleistet die hier wichtige Miniaturbauweise bei gleichzeitiger Robustheit der Geräte.

Sichere elektrische Kontakte zwischen den einzelnen Modulen der neuen Geräte auch nach Zerlegung und Zusammenbau, z.B. bei Wartungsarbeiten, sowie ebenfalls Raumersparnis, sind bei einer Ausführungsform gemäß **Anspruch 11** gegeben.

Schließlich ist es vom Gerätesicherheits-Standpunkt aus von besonderem Vorteil, eine wie vom **Anspruch 12** wiedergegebene, galvanische Trennung von Kameraversorgung und übrigen Leitungen und Funktionselementen und -organen sicherzustellen.

Eine besonders kostenschonend zu verwirklichende Ausführungsvariante, welche mit nur einer handelsüblichen problemlos wechselbaren Glühbirne ausgestattet ist und ein robustes optisches System sowie ein einfaches Aufnahmegerät aufweist, wird vom **Anspruch 13** umfaßt.

Die Verwendung nur einer Glühbirne, z.B. Halogentyp mit 10 mm-Fassung führt zum Entfall von Kalibrationsproblemen und ermöglicht einen einfachen, kostengünstigen Austausch. Bei Einsatz rotationssymmetrischer Bauteile wird jedenfalls eine Senkung der Produktionskosten erzielt.

Durch die technischen Merkmale gemäß **Anspruch 14**, insbesondere das Absetzen des Videochips in der Kamerahalterung wird ein besonders vorteilhafter komprimierter Aufbau gewährleistet. Als Stellorgan hat sich eine einfache Stellschraube besonders bewährt.

Besonders hautschonend und patientenfreundlich sind Ausgestaltungen der neuen Untersuchungseinrichtung gemäß den **Ansprüchen 15** und **16**.

Weiterer wesentlicher Gegenstand der vorliegenden Erfindung ist ein neues Verfahren zur optischen Untersuchung von Humanhaut, insbesondere zur reproduzierbaren Erkennung und Untersuchung von pigmentalen Hautläsionen, bevorzugt unter Einsatz einer wie oben beschriebenen neuen Hautuntersuchtungseinrichtung gemäß Oberbegriff und Kennzeichen des **Anspruches 17**.

Zur näheren Erläuterung der erfindungsgemäßen Untersuchungsmethodik sei besonders auf die Ausführungen zu **Anspruch 1** verwiesen.

Eine 3-Stufen-Sequenz-Technik gemäß **Anspruch 18** hat den Vorteil einer umfassenden Analyse und Diagnose, wobei der durch Immersionstechniken erzielbare Informationsgehalt bei weitem übertroffen wird, was zu besonders hoher Beurteilungs-Sicherheit beiträgt.

Eine besonders gleichmäßige Ausleuchtung einer zu untersuchenden Hautstelle und damit eine dann weniger kompliziertere Auswertung der Bildinformation ermöglicht eine Verfahrensausgestaltung gemäß **Anspruch 19**.

Für besonders spezialisierte Aufgaben bezüglich Aufklärung zu untersuchender Hautstrukturen eignet sich eine Verfahrensvariante gemäß **Anspruch 20**.

Möglichst geringe Patientenbelastung gewährleistet eine abstandgesteuerte, automatische Lichtabschaltung gemäß **Anspruch 21**.

Eine andere derartige, entlastende Beleuchtungsabschaltung betrifft **Anspruch 22**.

Von den mittels der bis jetzt beschriebenen Varianten des erfindungsgemäßen Verfahrens gelieferten Daten und Bildinformationsgehalten gespeist, hat sich ein die wesentlichen Schritte gemäß **Anspruch 23** umfassendes Auswerteverfahren als vorteilhaft erwiesen.

Bei einem insbesondere für Ausbildungszwecke vorteilhaften Einsatz des neuen Diagnosesystems im Rahmen einer Lehrroutine kann eine Beispielläsion am Schirm gezeigt und der Betrachter nach Diagnose und Kriterien befragt werden. Ein zweiter Schirm zeigt die Übereinstimmung zwischen dem vom Untersucher angegebenen Resultat und der Realität. Zum Beenden der Vergleichsoption kann jeder der dargestellten Schirme über ein Abbruch-Schaltfeld verlassen werden.

Das Editieren und die Datenpflege beinhalten die Optionen "Aufsuchen" und "Update" der Textdaten, bzw. "Aufsuchen" und "Löschen".

Eine Computer-unterstützte Diagnose pigmentierter Hautläsionen kann vorteilhaft grundsätzlich wie folgt, eingerichtet sein: Dabei ist eine prinzipielle Frage in der Auflichtmikroskopie essentiell: Ist die betreffende Läsion gutartig oder bösartig, d.h. soll die pigmentierte Läsion operativ entfernt werden oder nicht. Es ist heute aus der Literatur bekannt, daß die Sensitivität, also das richtige Erkennen bösartiger Läsionen rund 70% beträgt, besonders dann, wenn die betreffende Läsion sich in einem Frühstadium befindet. Experten weisen eine rund 15% bessere Sensitivität auf.

Aus all den genannten Gründen ist es äußerst wünschenswert ein System zur Verfügung zu haben, das die Diagnose von nicht erfahrenen Anwendern unterstützen und somit die Frühdiagnose von Melanomen verbessern kann. Da das neue Verfahren mit Video- und Computer-System Bilddaten digitalisiert, war die Entwicklung eines neuen Bildverarbeitungsmoduls zur Diagnosehilfe zielführend:

Die Funktion dieser Methode wird im folgenden kurz erläutert, wobei betont werden soll, daß die hier beschriebene Methode zur Diagnoseunterstützung tatsächlich auf einfachen Standardprozeduren der Bildverarbeitung beruht und daher jeweils nur kurz auf die Funktionsweise der einzelnen Prozeduren eingegangen wird. Wesentlich ist dabei eben die Abfolge und Wahl der einzelnen Algorithmen, bzw. die Wahl der extrahierten Features.

Eine den Gegenstand des **Anspruches 24** bildende, Fehlerquoten reduzierende, weitgehend automatisierte und damit objektivierte Objekterkennung mittels multiphasischer Segmentierung bzw. lokaler Schwellwertbildung sowie Bilddatenreduktion im Rahmen der vorliegenden Erfindung funktioniert im wesentlichen folgendermaßen:
- Die pigmentierte Läsion wird entweder durch multiphasische Segmentierung oder durch lokale Schwellwertbildung von der umgebenden Haut sicher getrennt. Dazu ist kein Farbbild notwendig. Es genügt ein Schwarzweißbild aus dem roten + grünen Kanal, z.B. des Videosystems. In der hier angewendeten Methode wird das Bild auf zehn Grauwerte reduziert, wobei die höchste Graustufe 10 die gesunde Haut und die niedrigste Graustufe 1 die dunkelste Stelle der Pigmentläsion repräsentieren. Es entsteht auf diese Weise ein zwar sehr grobes, jedoch im Umriß genaues Bild der Läsion und der umgebenden Haut. Besonderer Vorteil dieses Verfahrens ist, daß es nicht auf eine genaue Kalibierung der Belichtung angewiesen ist, da nur relative Größen verarbeitet werden.
- In einem Maskierungsschritt wird die zur Bilddatenreduktion gebrachte Läsion automatisch maskiert.
- Anhand eines Binärbildes der gesamten Läsion werden folgende Parameter bestimmt: Fläche, Umfang, Achsenverhalten, also Symmetrie, fractale Dimension der Begrenzung sowie Aspect ratio.
- Eine Beurteilung des Randes wird in folgender Weise vorgenommen: Da die Begrenzung einer Pigmentläsion sehr viel über ihre Dignität aussagen kann, wird der Rand so beurteilt, daß aus den Graustufen 9,8 und (8+9) ein Binärbild produziert wird, welches Binärbild auf Fläche in Relation zur Gesamtfläche, Umfang und fractale Dimension hin analysiert wird.
- Für eine Beurteilung der Farbe und Farbverteilung wird das Image vom RGB-Farbraum (Rot-Grün-Blau) in den HSI-Farbraum (Hue-Saturation-Intensivity) über Formeln transferiert. Am HSI-Bild repräsentiert nun ein per definitionem Schwarzweiß-Bild die Färbung der Objekte. Dieses Bild sowie die Varianz der Grauwertverteilung geben Auskunft über die Variabilität der Färbung.
- Es folgt eine Auswertung der Daten über ein neuronales Netzwerk auf folgende Weise: Durch die oben beschriebene Methode ist es zwar möglich, die Datenmenge eines Bildes deutlich zu reduzieren, trotzdem liegt eine große Zahl von Parametern vor, die sich einer Beurteilung durch den Diagnostiker infolge ihrer Fülle entziehen. Zur Auswertung können die verschiedensten multivarianten Verfahren herangezogen werden, um zu einer jeweils sinnvollen aussagekräftigen und auch sensitiven Klassifikation zu gelangen. Als am besten dafür geeignet hat sich die Applikation eines neuronalen Netzwerkes bewährt, das schon mit einigen hundert Trainingsdaten bekannter Diagnosen gemäß u.a. Schematik, konfrontiert worden ist. Dabei wird vorteilhaft der sogenannte "backpropagation"-Algorithmus verwendet. Auf diese Weise bereits trainierte Netzwerke können sogar auf PC-Basis mit Hilfe der genannten Daten innerhalb weniger Sekunden die zutreffende Diagnose stellen.

Dabei wird eine aktuelle Läsion in einer der möglichen Diagnoseklassen eingereiht: (1): unverdächtige Läsion; (2): verdächtige Läsion - Empfehlung einer Begutachtung durch Experten für Pigmentläsionen; (3): wahrscheinlich bösartige Läsion - Excision und histologische Aufarbeitung erforderlich.

Die mit einem solchen System bzw. Verfahren bis dato erzielte Sensitivität beträgt mehr als 70%, also mehr als das von Menschen erzielte Maß.

Mit Hilfe eines wie oben angegebenen Auswerte- und Diagnostikmoduls in der beschriebenen Weise kann tatsächlich eine Verbesserung der diagnostischen Treffsicherheit erzielt werden. Durch das Zusammenwirken von mit polarisiertem Licht arbeitenden Verfahren und Geräten, eine an sich einfache Archivierung der Pigmentläsionen, die Möglichkeiten Archivläsionen anhand ihrer Abbildungen im Vergleich zu betrachten, sowie eine Beurteilung von Pigmentläsionen durch die Maschine selbst, kann eine Früherkennung von malignen Melanomen deutlich verbessern und somit einen wesentlichen Beitrag zu einer Reduktion der Sterblichkeit an diesem inzwischen sehr häufigen Tumor leisten.

Was die bei den zur Erfindung führenden konkreten praktischen Untersuchungen eingesetzten Computer und Monitore betrifft, wird dazu folgendes ausgeführt:
Computer: Es wird ein handelsüblicher Computer vom Industriestandard (IBM kompatibel) verwendet. Grundvoraussetzungen sind ein Prozessor 386 und 486, mindestens 4 MB RAM, sowie 3 freie 16 Bit Slots. Die Festplatte sollte mindestens 250 MB aufnehmen können.
Monitor: VGA-Monitor, handelsüblich.

An zusätzlicher Hardware ist eine frame-grabber Karte notwendig, um die eingehenden Videosignale auch verarbeiten zu können. Diese frame-grabber Karten sind handelsüblich und werden unverändert verwendet. Es konnten zwei Produkte getestet werden.
1. Video-Blaster frame grabber unter Windows.
2. Mi(k)ro-Movie pro frame grabber unter Windows.

Die Software ist in Visual-Basic 3.0 unter Windows geschrieben und enthält folgende Features:
1) Patientendokumentation und Archivierung
2) Bilddokumentation und Archivierung
3) Befundausgabe

Um die Funktion der Sofware zu verdeutlichen, sei ein "Geschäftsfall" beschrieben. Es soll durch den speziellen Aufbau der Steuerungs- und Archivierungssoftware dem Bediener (Arzt) möglich sein, ohne Unterbrechungen für Eingaben über das keyboard des Computers, mehrere Läsionen digital aufzunehmen, zu speichern und der jeweiligen Lokalisation am Körper des Patienten zuzuordnen. Am Beginn einer auflichtmikroskopischen Untersuchung steht jedoch nicht fest, wieviele Läsionen begutachtet bzw. dokumentiert werden.

### Beschreibung des Ablaufs:

### 1. Beginn der Untersuchung (1.Schirm)

Aufnahme der Personaldaten des Patienten, (im Vergleich mit den vorhandenen Daten), Feststellen ob der Patient bereits einmal dokumentiert wurde. Jedes aufgenommene Bild (file) wird also mit einer unikalen Bezeichnung versehen, welche den Patienten eindeutig identifiziert und zudem noch einen Zähler enthält. Die Vergabe des Datums erfolgt automatisch über den internen Timer des Computers.

### 2. Übertragung des Signals der Videokamera on-line (2.Schirm) "live-video"

Durch die direkte Übertragung des bereits digitalisierten Signals von der Videokamera ist nun eine Betrachtung der Hautläsionen möglich. Diese direkte Betrachtung über den Monitor ist solange möglich, bis über den in der digitalen Auflichtkamera eingebauten Mikroschalter der Befehl zum Einfrieren (capturing) des Bildes gegeben wrid. Ein Schaltfeld am Schirm erlaubt es, die Läsion auf Platte zu speichern.

### 3. Identifikation der Lokalisation (3.Schirm) "Bildspeicherung-Lokalisation"

Der 2. Schirm blendet sich automatisch aus. Eine verkleinerte Abbildung des eingefangenen Bildes wird an der linken Bildschirmhälfte angezeigt. Am restlichen Bildschirm wird eine digitalisierte Abbildung eines Menschen von vorne frontal und dorsal gezeigt. Mittels eines in das digitale Auflichtmikroskop eingebauten Trackballs als elektronisches Zeigeinstrument ist es nun möglich, die Lokalisation der Läsion anzusteuern und durch Druck auf einen Mikroschalter zu fixieren. Dabei werden die Koordinaten dieses Punktes dem Bildfile zugeordnet und abgespeichert.

### 4. Wiederholroutine (Schirm 2-3)

Ein Schaltfeld am Schirm 3 ermöglicht die Wiederholung des Darstellungs- und Speicherprozesses, um weitere Läsionen darzustellen und unter Umständen zu dokumentieren.

### 5. Zusätzliche Optionen

### Bildergalerie des identifizierten Patienten, "thumbnails"

Der Wert der digitalen Auflichtmikroskopie unter Einsatz der Polarisationseinrichtung(en) gründet sich besonders auf der Tatsache der Darstellung einer Läsion zu zwei oder mehr verschiedenen Zeitpunkten. Damit wird es möglich, dynamische Aussagen über eine Läsion zu treffen und damit Entscheidungen über das weitere Schicksal des Pigmentmals zu fällen, wie z.B. Entwicklung eines Melanoms aus einem dysplastischen Nävus.

Wurde ein Patient erfolgreich identifiziert, werden verkleinerte Abbildungen aus dem Bildarchiv am Bildschirm dargestellt. dazu wird jeweils die Läsion und unmittelbar benachbart die Lokalisation dargestellt. Durch Anklicken kann dieses Bilderpaar vergrößert werden (Schirm 3). Ein Schaltfeld ermöglicht die Option eines Vergleiches der Läsionen zu verschiedenen Zeitpunkten. Die Darstellung erfolgt von links nach rechts mit jeweils 4 Bilderpaaren pro Schirm. Die Anzeige erfolgt chronologisch zum Zeitpunkt der jeweiligen Untersuchung. Zusätzlich werden die Bilder nach dem jeweiligen Zuordnungszustand geordnet dargestellt.

Zum Vergleich von Läsionen zu zwei verschiedenen Zeitpunkten folgendes: Nach Aktivierung eines Bilderpaares von Läsion und Lokalisation wird ein Schaltfeld angeklickt, welches den Vergleich zu zwei verschiedenen Zeitpunkten ermöglicht. Dabei wird der Schirm geteilt, die Lokalisationsinformation (Bild links) ausgeblendet und das live-video Fenster rechts eingeblendet. Es ist somit möglich, ein und dieselbe pigmentierte Läsion einmal aus dem Archiv darzustellen und simultan mit dem rezenten Bild zu vergleichen. Über den Mikroschalter am digitalen Auflichtmikroskop ist es möglich, die Abbildung einzufangen. Dabei wrid automatisch abgespeichert, ohne die Lokalisation nochmals abzufragen, da angenommen werden darf, daß beide Lokalisationen exakt übereinstimmen. Zusätzlich wird ein Zeiger im file abgelegt, der Information über die Verknüpfung der Abbildungen zu zwei oder mehreren verschiedenen Zeitpunkten gibt.

Was die Darstellung von verknüpften Abbildungen betrifft, wird folgendermaßen vorgegangen: Durch den Zeiger im file ist es möglich, miteinander verknüpfte Abbildungen zu identifizieren. In der Darstellungsroutine werden die Läsionen als thumbnails dargestellt. Als Quelle dient die jweils am längsten zurückliegende Läsion. Durch Anklicken dieses thumbnails ist es möglich, die Abbildungen dieser Läsion in einer Zeitreihe darzustellen (wieder als thumbnails) und durch weiteres Anklicken bzw. Aktivieren in die Routine des Vergleiches von Läsionen zu verschiedenen Zeitpunkten zu gelangen.

Schließlich ist weiters die Option Datenfernübertragung, mailbox und Beurteilung durch Experten zu erwähnen. Die Option Datenfernübertragung dient zur Unterstützung der Anwender in besonders schwierigen Fällen. Dabei wird eine schwierig zu beurteilende Läsion digital an eine mailbox gesendet, um dort von Experten beurteilt zu werden. Die Datenübertragung erfolgt über ein handelsübliches Modem, als Übertragungssformat der Information wird das komprimierte JPEG-Format herangezogen. Die Größe des files im JPEG-modus beträgt rund 50 kByte, die dafür notwendige Zeit zur Datenübertragung ist somit vertretbar. Die Software beinhaltet einen eigenen Menüpunkt zur Datenfernübertragung. Die gewünschte Läsion wird dabei mittels Maus angeklickt, die Ansteuerung des Modems erfolgt automatisch. Ein zusätzliches Datenfeld erlaubt, Textinformation zu übertragen.

Hinsichtlich einer Option "Lemen an Beispielen", also eines Fall-basierten Konsultationssystems ist auszuführen, daß ein essentieller Teil des diagnostischen Prozesses in der Auflichtmikroskopie der Vergleich mit bereits geklärten Fällen ist. Dazu war bis dato immer das Gedächtnis des Untersuchers, sowie dessen Abstrahierungsfähigkeit notwendig. Ein neuer optischer Eindruck, eine neue pigmentierte Läsion wird mit bereits bekannten Fällen im Gehirn verglichen. Für den geringer erfahrenen Anwender stellt somit ein Bildvergleichssystem eine wesentliche Erleichterung dar, welches einerseits die fragliche neue Läsion, andererseits bereits analysierte und diagnostizierte Fälle darstellt und erläutert. Im System sind bereits rund 200 Pigmentläsionen (10 Mbyte) gespeichert (zu jeder Pigmentläsion ist das Bild, die Diagnose sowie die Auflistung der auflichtmikroskopischen Kriterien vorhanden).

Der Ablauf des Vorgangs erfolgt auf folgende Weise.
- Aktivieren des fraglichen Bildes
- Aktivieren der Vergleichsoption

Das fragliche Bild wird verkleinert dargestellt, wobei folgende drei Modi zur Diagnoseunterstützung zur Verfügung stehen:

### Blättern in den dokumentierten Beispielläsionen nach Diagnose:

Der Untersucher wird nach einer Verdachtsdiagnose befragt - die Maschine zeigt auf der rechten Schirmseite passende Beispielläsionen. Es kann somit an bereits diagnostizierten Beispielen ein optischer Vergleich (mit der immer sichtbaren fraglichen Läsion) getroffen werden. Alle Beispielläsionen sind bezüglich Diagnose und auflichtmikroskopischer Kriterien in einem Textfeld dokumentiert.

Blättern in den dokumentierten Beispielläsionen nach auflichtmikroskopischen Kriterien: Da der Diagnosegang der Auflichtmikroskopie auf der Musteranalyse auflichtmikroskopischer Kriterien erfolgt, wird der Untersucher nach jenen Kriterien befragt, die er als Beispiel sehen möchte. Es werden dann jene Bilder gezeigt, die diese Kriterien enthalten. Wieder kann ein sofortiger Vergleich mit der fraglichen Läsion getroffen werden.

"Atlas": Die gespeicherten Beispielläsionen werden automatisch gezeigt und mit den zugeordneten Textfeldern (Diagnose, zur Diagnose führende Kriterien) erläutert.

Anhand der Zeichnung wird die Erfindung näher erläuter.

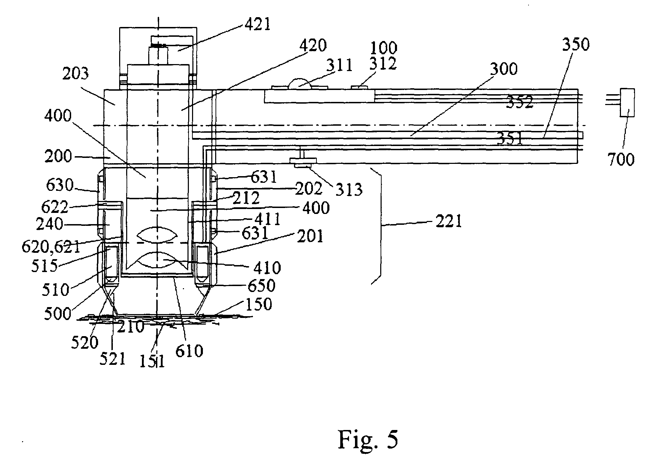

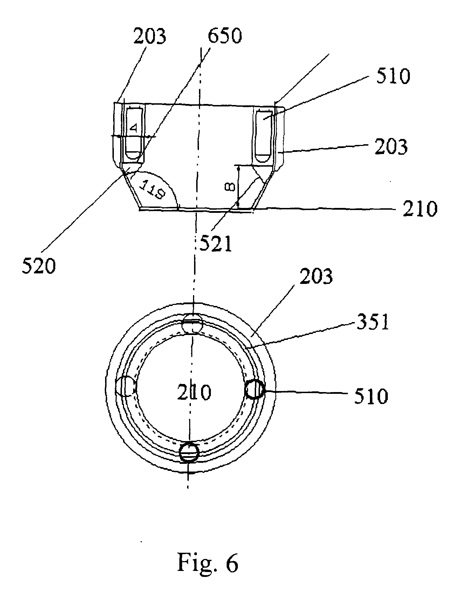

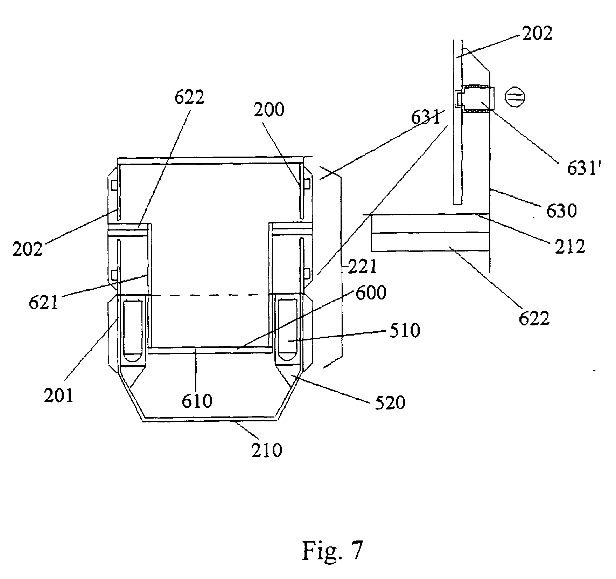

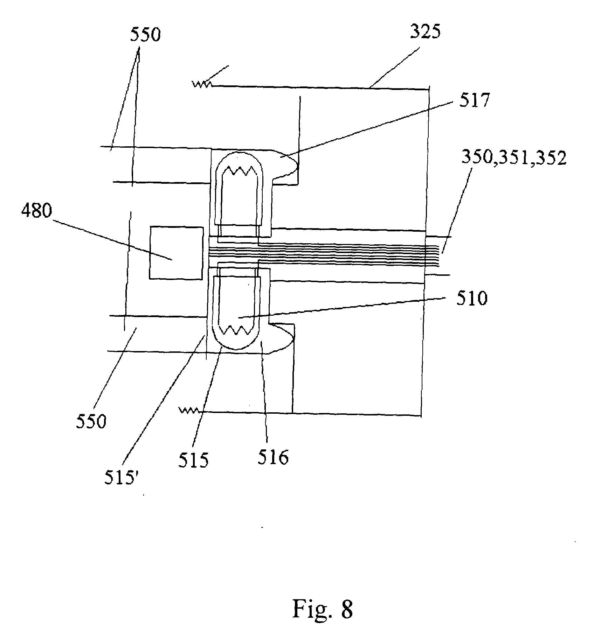

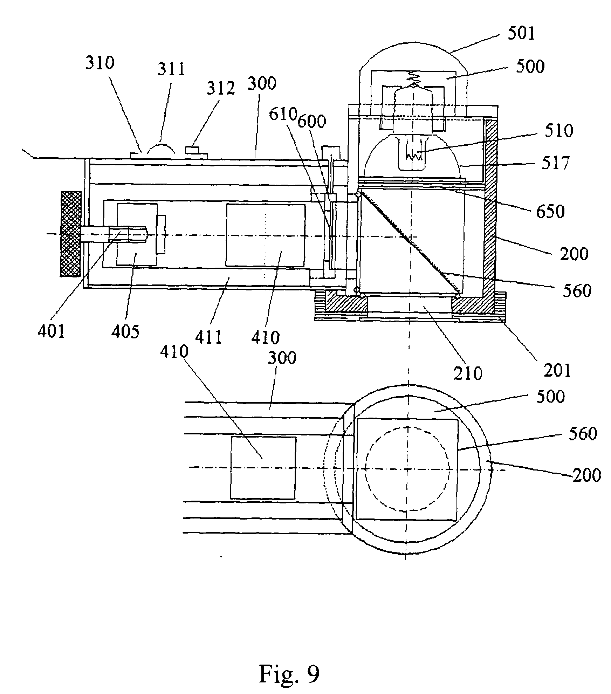

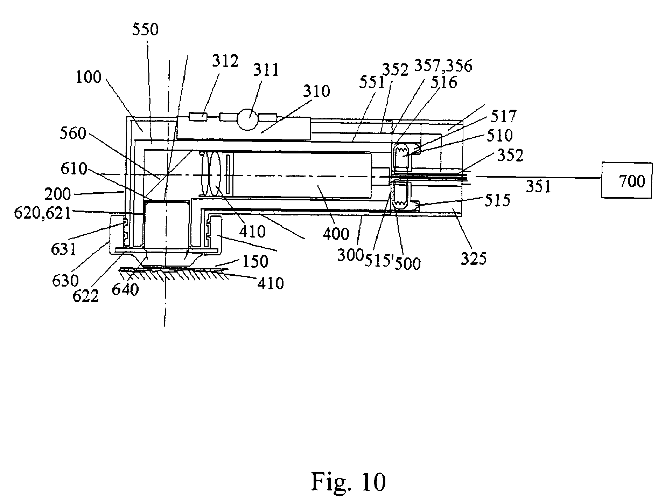

Es zeigen die Fig. 1 bis 4 jeweils eine schematische Schnittansicht von Humanhaut mit schematischen Darstellungen der bisher angewandten und der erfindungsgemäßen Techniken, die Fig. 5 die Schnittansicht einer bevorzugten Ausführungsform der erfindungsgemäßen Untersuchungseinrichtung, Fig. 6 eine schematische Frontal-Ansicht des Gehäusevorderteils des Gerätes gemäß Fig. 5, Fig. 7 ein schematisches Detail der Gleitdreh-Lagerung des einen Polarisationsanalysator tragenden Gehäuse-Überwurfzylinders, an einem Gerät gemäß Fig. 5, Fig. 8 die Schnittansicht einer weiteren, vorteilhaften Ausführungsvariante der erfindungsgemäßen Untersuchungseinrichtung, Fig. 9 eine schematische Detailansicht einer dislozierten Beleuchtungseinrichtung, wie sie vorteilhaft für ein Gerät gemäß Fig. 8 vorgesehen ist und der schematische Schnitt und die schematische Draufsicht der Fig. 10 repräsentieren eine besonders robuste und kostengünstig zu produzierende Variante der neuen Untersuchungsgeräte.

Die Fig. 1 stellt schematisch eine einfache Betrachtung der Haut ohne auflichtmikroskopische Techniken anhand eines stark vergrößerten Vertikalschnitts durch menschliche Haut 150, an deren Beobachtungsstelle 151 dar. Die vollen Kreise stellen die Pigmentzellen oder Melanozyten 156, welche Pigmentnester 157 bilden, dar. Die Anordnung dieser Pigmentzellen 156 ist für den Krankheitsprozeß an pigmentierten Hautläsionen charakteristisch. Drei wesentliche physikalische Grenzschichten sind von Bedeutung: a) Grenzschicht zwischen Luft und der Hornschicht 152, b) Grenzschicht 154 zwischen Oberhaut-Epidermis 153 und Unterhaut-Dermis 155, c) Grenzschicht zwischen den pigmentierten Zellen 156 in der Haut 150 und ihrem umgebenden Medium. Die Dicke d der Oberhaut-Epidermis beträgt rund 0,1 - 0,3 mm.

Die Skizze stellt den einfallenden Lichtstrahl dar. Der größte Anteil des Lichts wird bereits an der obersten Grenzschicht zwischen Luft und Hornschicht 152 diffus reflektiert, hier ist der entsprechende Lichtstrahl mit bezeichnet. Ein weitaus geringer Anteil wird von den anderen Grenzschichten reflektiert. Der größte Anteil des eintreffenden Lichtes a wird in den oberen Anteilen der Unterhaut-Dermis 155 absorbiert.

Ohne Anwendung auflichtmikroskopischer Techniken ist es deshalb nicht möglich, in die Oberhaut-Epidermis 153 einzusehen, da der größte Anteil des Lichtes reflektiert wurde und an Quantität den eindringenden Lichtanteil weit überwiegt, so daß zum Beobachter 40 nur wenig Licht aus diesen Zonen gelangt, welches zudem noch von der Hautoberflächen-Reflexion völlig überstrahlt wird.

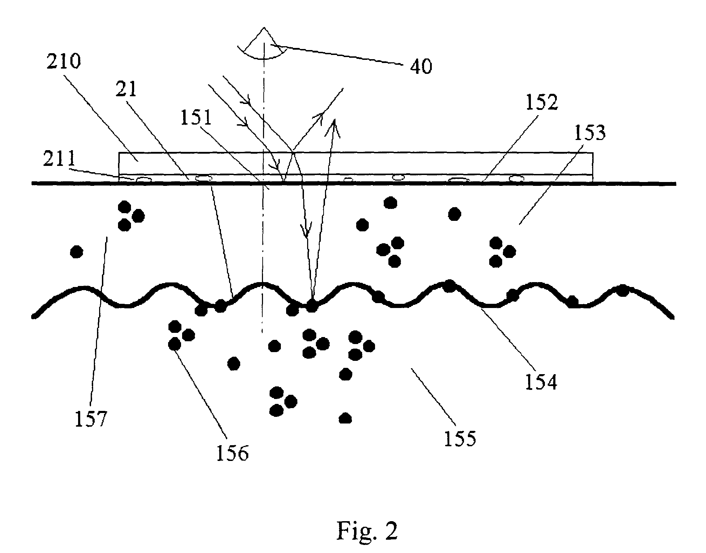

Die Fig. 2 symbolisiert - bei zu Fig. 1 völlig analogem Bezugszeichengebrauch - eine Betrachtung der Haut mittels konventioneller auflichtmikroskopischer Techniken. Die Aufbringung einer planen Glasplatte 210, sowie eines Tropfens Immersionsöl 21 unter dieser Glasplatte 210 reduziert den diffus reflektierten Anteil des Lichtes an der obersten Grenzschicht. Es kann nun relativ mehr Licht reflektiert aus den tieferliegenden Anteilen der Epidermis 153 wahrgenommen werden. Der wesentliche Nachteil dieser Methode erklärt sich aus dem Benetzen der Hautoberfläche mit Öl 21 und deren störenden Reflexionen, sowie der Entstehung störender Luftblasen 211 im Ölfilm 21, wo ja neue Grenzschichten zwischen optisch dünnen und dichteren Medien auftreten, welche eine Beobachtung einer zu untersuchenden Hautstelle 151 wesentlich beeinträchtigen.

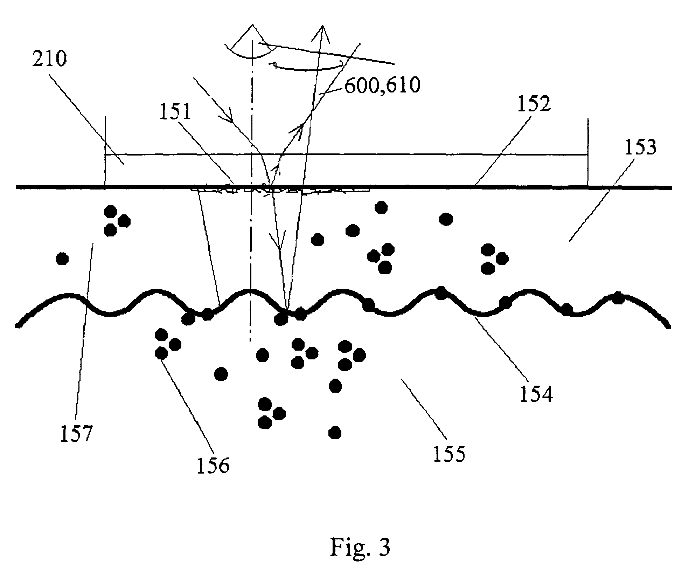

Die Fig. 3 zeigt, ebenfalls unter Verwendung von zu gemäß Fig. 1 analogen Bezugszeichen, die erfindungsgemäß vorgesehene Methode einer Betrachtung der Haut 150 mittels der Anwendung eines Polarisationsfilters 600,610, und zwar als Analysator, ohne die Verwendung von Immersionsöl. Licht strahlt auf die Hautoberfläche 151 ein. Der an der ersten Grenzschicht 152 reflektierte Anteil ist dann linear polarisiert und kann gemäß der Polarisationsebene eines Polarisationsfilters 600,610 (Analysator) - vor der Objektivebene eines Aufnahmegerätes oder vor dem Auge liegend - , ausgefiltert werden. Es ist dadurch möglich, ohne die Anwendung von Immersionsfluid, den damit erreichten auflichtmikroskopischen Effekt dennoch zu erreichen. Durch mit Pfeil angedeutetes Drehen des Analysators 600,610 ist es möglich, beide Arten der Darstellung, einerseits die konventionelle Lupendarstellung zur Beurteilung der Oberfläche, andererseits die auflichtmikroskopische Darstellung in einer Sitzung zu erzielen. Dieses Verfahren ermöglicht jedoch nicht eine relative Verstärkung des Bildes des Melaninpigmentes 156, 157, da nur ein Polfilter 600, 610 verwendet wird. Für eine Routineapplikation ist dieses Prinzip jedoch durchaus sinnvoll, da relativ mehr Licht für die Betrachtung einer Pigmentläsion zur Verfügung steht.

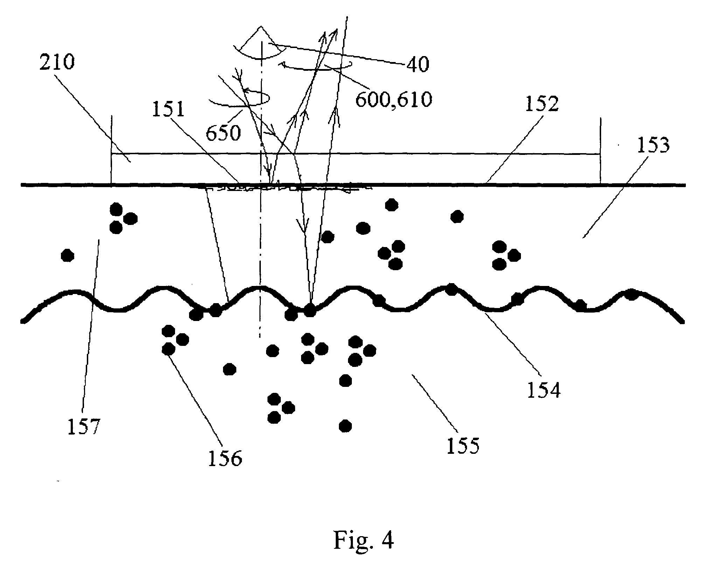

Die Fig. 4 zeigt, wieder unter Verwendung von zu gemäß Fig. 1 analogen Bezugszeichen, eine erfindungsgemäße Betrachtung der Haut mittels Anwendung von zwei Polarisatoren 600,650 ohne Verwendung von Immersionsöl. Mit 650 linar polarisiertes Licht strahlt auf die Hautstelle 151 ein. Der an der ersten Grenzschicht 151 reflektierte Anteil ist nun auch linear polarisiert und kann gemäß der Polarisationsebene eins zweiten Polarisationsfilters bzw. Analysators 600 vor der Objektivebene bzw. dem Auge 40 liegend, ausgefiltert werden. Es ist dadurch möglich, ohne die Anwendung von Immersionsöl den auflichtmikroskopischen Effekt zu erreichen. Durch Drehen des zweiten Polarisationsfilters 600 ist es zudem möglich, beide Arten der Darstellung, einerseits die konventionelle Lupendarstellung zur Beurteilung der Oberfläche, andererseits die auflichtmikroskopische Darstellung in einer Sitzung zu erzielen. Da das in den Pigmentzellen-Melanozyten 156, 157 enthaltende Pigment eine optisch aktive Substanz darstellt, läßt sich durch geeignete Position des Analysators 600 der optische Eindruck dieser Zellpopulation noch verstärken.

Das in Fig. 5 gezeigte, kleindimensionierte Gerät 100 zur Untersuchung von Humanhaut 150 an einer gewünschten Stelle 151 weist ein mit dem eine Frontplatte 210 aus Transparentmaterial aufweisenden, vorderen Gehäuseteil 201, dem fluchtend ihm anschließenden, hinteren Gehäuseteil 202 (die beiden Gehäuseteile 201, 202 bilden zusammen einen Gehäusedrehteil 221 und dem Gehäusebasisteil 203 gebildetes Gehäuse 200 mit sich von diesem im Bereich der Gehäusebasis 203 im rechten Winkel radial wegerstreckendem Gehäusefortsatz 300 auf, durch welchen mittels Schaltorganen 310 mit Trackball 311, Mikroschalter 312 und Mikrotaster 313 steuer- und schaltbare Versorgungs-, Daten-, Signal- und Steuerleitungen 350,351,352 geführt sind, von wo Versorgungsleitungen 351 zu einem optischen Aufnahmegerät 400 und einer Beleuchtungseinrichtung 500 im Gehäuse 200 und mindestens eine Steuerleitung 352 zu einer externen Steuer-, Bildwiedergabe-, Bildspeicher-, Dokumentations-, Archivierungs- und/oder Diagnoseerstellungseinrichtung, vorzugweise zu einem für mindestens eine der genannten Funktionen programmierten Computer 700 mit Bildschirm oder Display, geführt sind.

Zentral und axial im Gehäuse 200 ist als Aufnahme- und Beobachtungsgerät eine Mikrovideokamera 400 mit Objektivteil 410 im Objektivgehäuse 411, Kameraträger 420 und das Gehäuse 200 überragendem Kameraabschluß 421 untergebracht. Im vorderen Gehäuseteil 201 ist im Ringraum 240 zwischen der Wandung des Gehäuseteils 201 und dem Objektivgehäuse 411 eine mehrere Lichtemissionselemente, insbesondere Mikroglühtampen 510, und eine dieselben tragende Beleuchtungshalterung 515 aus transparentem Kunststoffmaterial umfassende Beleuchungseinrichtung 500 angeordnet. An dieselbe schließt gegen die optische Öffnung mit der Transparentplatte 210 hin ein Ringprisma 520 mit als Licht-Diffusor dienender aufgerauhter, schräger Innenfläche 521 an, welches an seiner Oberseite fakultativ oder fakultativ schaltbar eine Polarisatorfolie bzw. eine steuerbare Polarisationseinrichtung 650 aufweist, die gewünschtenfalls zur Erzeugung von in jeweils gewünschter Ebene schwingendem, auf eine zu untersuchende Hautstelle 151 einzustrahlendem Licht dient. Um nun das ein Bild von der Hautstelle 151 liefernde, von derselben reflektierte Licht, das zumindest in Anteilen polarisiert ist, vor Eintritt in das Vergrößerungsobjektiv 410 der Videokamera 400 zu analysieren, gezielt zu schwächen od.dgl, ist vor dem genannten Objektiv eine Polarisationsfolie 610 einer Polarisationseinrichtung 600 angeordnet, welche von einem das Kameraobjektivgehäuse 411 umgebenden, zylindrischen Vorsprungteil 621 eines Polarisationsfilter- bzw. Analysator-Halterungs- und Drehorgans 620 gehalten ist, von welchem Vorsprungteil 621 sich - hier im rechten Winkel - ein Ringteil 622 durch einen Zwischenraum 212 zwischen vorderem 201 und hinterem Gehäuseteil 202 nach außen wegerstreckt, welcher peripher von einem die beiden genannten Gehäuseteile 201,202 übergreifenden, eine drehmanipulationsfreundlich gestaltete Außenfläche aufweisenden Überwurfzylinder 630 gehalten ist, welcher seinerseits an den Außenseiten der beiden zylindrischen Gehäuseteile 201,203 jeweils mit einer Art Nut- und Federkonstruktion 631 winkelverdrehbar gleitend gelagert ist.

In der gezeigten konkreten Ausführungsform hat das Gehäuse 200 mit herausragendem Kameraabschluß 421 eine Höhe von insgesamt 81 mm und der Gehäusefortsatz 300 eine Gesamtlänge von 131 mm.

Die Fig. 6 zeigt, bei sonst gleicher Bezeichnungsweise, wie in Fig.5, im Detail, wie im Gehäusevorderteil 201 mit Frontplatte 210, peripher an die Innenwand anschließend, eine im wesentlichen hohl-zylinderförmige Beleuchtungshalterung 515, z.B. aus transparentem Kunststoff, angeordnet ist, in deren vier gleichmäßig verteilten Ausnehmungen vier 4,5 V Halogen-Kleinstglühbimen 510 sitzen. An die Beleuchtungshalterung 515 schließt nach unten hin fluchtend ein ringförmiger Auflicht-Polarisator 650 und ein Ringprisma 520 mit rauher Licht-Abstrahlfläche 521 an. Aus der Unteransicht ist die Verkabelung 351 der elektrischen Glühlampen-Versorgung ersichtlich.

Das in Fig. 7 gezeigte Detail der Lösung der "drehbaren" Befestigung des Überwurfzylinders 630 der Analysatoreinrichtung 600 zeigt die Nut-Feder-Konstruktion der Drehgleitlagerung 631 des Überwurfzylinders 630 an der Außenseite der Gehäuseteile 201,202, wobei hier als "Feder" eine einfache Madenschraube 631' dient. Die sonst in dieser Figur aufscheinenden Bezugszeichen benötigen keine weiteren Erklärungen, sie sind jenen der Fig. 5 analog verwendet.

Das neue Haut-Untersuchungsgerät 100 in der Variante gemäß Fig. 8 unterscheidet sich im Prinzip nur wenig von jenem gemäß Fig. 5, es weist ein besonders "niedriges", vertikales Gehäuse 200 auf, und im - ebenfalls als Handgriff gestalteten - Gehäusefortsatz 300 ist hier das optische Aufnahmegerät, also konkret eine Videokamera 400 mit Objektiv bzw. Vergrößerungsobjektiv 410 und eine Beleuchtungseinrichtung 500 mit radial angeordneten Kleinstglühbirnen 510 in Ausnehmungen 516 einer transparenten Kunststoffhalterung 515 mit Lichtstrahl-Parallelricht-Reflektoren 517 untergebracht. Der die Beleuchtungseinrichtung 500 beinhaltende Gehäusefortsatz-Endteil 325 ist mit dem Restteil des Gehäusefortsatzes 300 lösbar verbunden und weist für zumindest die Schalt- und Steuerleitungen 352 zu dem Schalt- und Steuerorgan 310 mit Trackball 311 und Mikroschalter 312 Kontaktstifte 357 auf, welche mit entsprechenden Kontaktelementen 356 des restlichen Gehäusefortsatzes 300 leitungsverbindend kooperieren. Die Steuerleitungen 352 führen zu einer Steuer-, Schalt-, Bildsignal- Reduktions,- Verarbeitungs-, Wiedergabe-, Speicher-, Dokumentations- und/oder Archivierungseinrichtung 700, insbesondere Computer mit Bildschirm, Display, Lautsprecher od.dgl. Die Endfläche 515' der Beleuchtungshalterung 515 schließt satt an die hier rohrartig mit Knick in das Gehäuse 200 sich hineinerstreckenden Lichtleit- und - emissionseinrichtung 550, z.B. aus mit Reflexionsmaterial 551 gemanteltem Kunststoff, insbesondere Plexiglas, an.

Für die Umlenkung des Hautstellenbildes in die Videokamera 400 ist am Übergang vom Gehäusefortsatz 300 in das Gehäuse im Umlenkreflektor 560 angeordnet.

Für den Polarisations-Analysator 600 ist eine Analysator-Dreh- und Halterungseinrichtung 620 vorgesehen, welche mit der Transparentplatte 410 das Gehäuse 200 zur Haut 150 hin abschließt. Die Platte 410 steht in Zusammenhang mit einem wieder über Nut/Feder 631 an der Gehäuse-Außenseite drehgleitgelagerten Überwurfzylinder 630, dessen Ringteil 622, scheibenartig ausgebildet, einen nach unten gerichteten Blendschutz 640 und einen nach oben gestülpten, rohrartigen Vorsprungteil 621 aufweist, an welchem die Analysator-Polarisationsfolie 610 befestigt ist.

Die sonstigen in der Figur aufscheinenden, hier nicht näher erläuterten Bezugszeichen sind zu jenen gemäß Fig. 5 analog verwendet.

Die Fig. 9 zeigt - bei zu Fig. 8 analoger Bezeichnungsweise - die im vom nicht gezeigten Gehäuse schraublösbaren Gehäusefortsatz-Endteil 325 untergebrachte Beleuchtungseinrichtung 500 mit zentralen Versorgungs- und Steuerleitungen 350-352, transparentem Träger 515 mit Ausnehmungen 516 für federgehalterte Glühbirnen 510. Die Endfläche 515' schließt satt und ohne Lichtverlust an den rohrartigen Lichtleiter 550 an, in dessen Innerem der Adapter 480 der hier nicht gezeigten Vidokamera untergebracht ist. Im freibleibenden Raum hinter der Beleuchtungseinrichtung kann z.B. ein Teil der Zeigegerät- und Steuerelektronik untergebracht sein.

Gemäß der anfänglich beschriebenen, alten Technik wird Immersionsöl zur Reduktion des von der Hautoberfläche reflektierten Lichtanteils verwendet. Das gleiche Prinzip läßt sich, wie gezeigt, sozusagen mit Hilfe von doppelt polarisiertem Licht erreichen. Es kann faktultativ vor der Austrittsöffnung der Lichtquelle ein Polarisationsfilter angebracht oder eingeschaltet werden, und vor der Eintrittsöffnung des Objektives eines Aufnahmegerätes ist ein drehbares Polarisationsfilter angebracht. Die Menge des nun in das Objektiv eintretenden Lichtes ist somit regelbar. Der durch Reflexion polarisierte Lichtanteil ist durch Filtern auszuschalten. Diese Anordnung ermöglicht es, die pigmentierte Hautläsion mit dem und ohne den bekannten ELM-Effekt zu betrachten. Versuche mit einem Prototyp haben gezeigt, daß durch diese Technik der ELM-Effekt nicht nur erreicht, sondem an Qualität wesentlich übertroffen werden kann.

Ergänzend seien zu Aufbau und Funktionsweise der Geräte gemäß Fig. 5 und 8, bzw. von deren Aufnahmeeinheit nach funktionellen Aspekten noch folgende Bemerkungen angebracht:

Die Aufnahmeeinheit 400 umfaßt eine Videokamera mit Mikroobjektiv, welche über ein Kabel ein Videosignal liefert. Über ein mitgeführtes Kabel wird die Aufnahmeeinheit mit Strom versorgt.

Der Analysator 600 bzw. sein Polarisationsfilter 610 dient zur Ausnutzung des beschriebenen physikalischen Effektes der Ausschaltung störender Reflexionen. Der Analysator befindet sich im Gehäuse 200 und ist von einem zylindrischen Vorsprungteil 621 gefaßt. Eine Konstruktion mittels Überwurfzylinder 630 gestattet es, den Vorsprungteil und damit das Analysatorfilter 610 beliebig zu drehen.

Der vordere Gehäuseteil 201 dient als Abstandshalter und damit dazu, den korrekten Focus zum lichtempfindlichen Element bzw. Sensor 400 einzuhalten. Eine Gewindekonstruktion hoher Steigung erlaubt es, den Focus nachzujustieren. Die Abschlußglasplatte 210 hält das aufzunehmende Objekt, Haut 150, plan und verhindert das Eindringen von Fremdkörpern in das Gerät. Die Abschlußglasplatte 210 ist aus hygienischen Gründen austauschbar konstruiert.

Die Beleuchtungseinrichtung 500 sorgt für die Ausleuchtung der Betrachtungsfläche 151. Als Lichtquellen dienen handelsübliche Halogenglühbirnen 510 in Kleinstbauweise. Diese Beleuchtungseinrichtung 500 kann sowohl axial, also im Gehäuseteil 200 als auch im Gehäusefortsatz 300 mit Handgriff unter Lichtleitung 550 untergebracht sein. Fakultativ kann in beiden Fällen das emittierte Licht bereits polarisiert abgestahlt werden.

Eine Lichtsteuerung kann zur Schonung der Lichtquellen und zur Verringerung der thermischen Belastung der Patientenhaut vorgesehen sein: Eine permanente, schwache Lichtquelle emittiert Licht; ein Fototransistor mit Schaltung erkennt das Annähern an die Patientenhaut, bzw. der Kontakt der Glasplatte führt zur Reflexion des LED-Lichtes und schaltet die Stromzufuhr der Lampen.

Eine zweite Variante kann folgendes vorsehen: Eine Schaltung ist an die serielle Mausleitung eingebunden und erkennt Signale vom Trackball 411 oder Mikroschalter 412, eine elektronische Zeitschaltung schaltet die Stromzufuhr aus, wenn keine Mausbewegungssignale einlangen, also Inaktivität bei der aktuellen Untersuchung vorliegt.

Im Handgriff 300 kann ein Zeigegerät für die serielle Computerschnittstelle untergebracht sein. Es ist somit möglich, das Programm mit dem Auflichtmikroskop zu bedienen.

Ein vorteilhaft einsetzbares Computerprogramm zur "live"-Betrachtung, Speicherung und zum Vergleich der aufgenommenen Bilder zu verschiedenen Zeitpunkten erhält die Bildinformation über eine Videodigitalisierungskarte. Das Programm ist in der Weise konzipiert, die Signale des Zeigegerätes zu verarbeiten, um dem Arzt die umständliche Bedienung des üblichen Keyboards zu ersparen. Alle Funktionen - mit Ausnahme der Eingabe von Personaldaten - können vom Auflichtgerät fernbedient werden.

Wenn ein Computerprogramm zur selbständigen Beurteilung der aufgenommenen Pigmentläsionen vorgesehen ist, kann unter Anwendung von Methoden der Bildverarbeitung eine Pigmentläsion als Objekt des Interesses automatisch erkannt, nach verschiedensten Kriterien analysiert und mit Daten aus einem neuronalen Netzwerk verglichen werden. Dadurch ist es möglich, eine computergestützte Klassifikation bzw. Diagnose zu erzielen.

Die einzelnen konkreten Spezifikationen sind bevorzugt folgende:

Auflichtmikroskopisches Gerät: Kamerahub +/- 2,5 mm; Drehbereich des Analysators: > 180°; effektives Gesichtsfeld: 10 mm; Lichtquellen: 4 x 1,5 V oder 4,5 V Glühlampen (Baumaß 5 x 3 mm); Frontscheibe: Mineralglas; Computerprogramme: Programmiersprache: Visual Basic bzw. C oder C++.

Was die konkret verwendete Videokamera betrifft, ist folgendes auszuführen:

Im Einsatz hat sich eine Kleinst-Videokamera von Panasonic mit den Baumaßen: Durchmesser 15 mm, Baulänge 25 mm besonders bewährt. Das Auflösungsvermögen beträgt 780 x 640 Pixels, als Signal steht PAL zur Verfügung. Alle Videokameras, die zumindest diese baulichen und technischen Auflagen erfüllen, sind einsetzbar. Die Netzversorgung der Kamera ist galvanisch vom restlichen System getrennt.

Das Objektiv ist ebenfalls handelsüblich und erzielt ein Gesichtsfeld von etwas mehr als 10 mm im Durchmesser. Das somit resultierende Gesichtsfeld ist kleiner als das Objektivgesichtsfeld und verwendet nur die zentralen, optisch hochwertigen Anteile des Objektives.

Die in Fig. 10 in Schnittansicht und Draufsicht gezeigte kostengünstige Variante einer erfindungsgemäßen Einrichtung weist ein - hier hohlzylindrisches - relativ kurz gebautes Gehäuse 200 mit es nach unten begrenzender von einem Steckkörper 201 abschließender Glasplatte 210 auf, von welchem sich im rechten Winkel ein außen bevorzugt als Manipulationsgriff ausgebildeter, mit Schalt- und Steuerorgan 310, mit Trackball 311 und Mikroschalter 312 für Schaltung und Steuerung einer nicht gezeigten externen Steuer-, Wiedergabe- und/oder Speichereinrichtung, insbesondere Computer ausgestatteter, hier ebenfalls hohl-zylindrischer, Gehäusefortsatz 200 wegerstreckt. Im geometrischen Durchdringungsraum von Gehäuse 200 und Fortsatz 300 ist unmittelbar hinter der Glasplatte 210 als Lichtleit- und -umlenkorgan ein zweiteiliges Würfelprisma 560 und im Gefolge der optische Achse des Gehäuses 200 entlang ein - hier fixer - Polarisator 650 und eine Beleuchtungseinrichtung 500 mit Gehäuse 501 und Glühbirne 512 sowie Parabolreflektor 517 angeordnet. Entlang der optischen Achse des Gehäusefortsatzes 300 sind, vom Prisma 560 weg, ein drehbarer Analysator 600 mit Polarisationsfolie 610 und in einer Halterung 411 eine Objektivoptik 410 sowie ein mittels Stellschraube 401 focuseinstellbarer Videosensor 405 einer optischen Aufnahmeeinheit 400 angeordnet.

Diese Bauvariante zeichnet sich insbesondere durch ein Umlenkprisma aus. Durch dessen Einsatz ist eine orthogonale Beleuchtung möglich, während die Betrachtung durch eine normal auf die Beleuchtungsrichtung abgesetzte Kameraeinheit erfolgt. Der Aufbau ist prinzipiell wie bei den bereits in den vorhergehenden Figuren gezeigten Varianten.

## Patentansprüche

1. Einrichtung zur optischen Untersuchung von Humanhaut und deren Pigmentierungen mit einem zylinderartigen Gehäuse, in welchem eine - eine optische Vergrößerungseinrichtung aufweisende - optische Beobachtungseinrichtung sowie eine Auflicht-Beleuchtungseinrichtung angeordnet sind, wobei im Strahlengang des von der Beleuchtungseinrichtung (500) abgestrahlten, auf eine aktuell zu untersuchende Hautstelle (151) gerichteten Lichtes und zusätzlich oder alternativ im Strahlengang des von der Haut (150) reflektierten Lichtes eine Polarisationseinrichtung, mit Polarisator (650) und/oder Analysator (600) vorgesehen ist und sich vom Gehäuse (200) ein hohler, als Manipulationsgriff ausgebildeter Gehäusefortsatz (300) wegerstreckt, durch welchen elektrische Versorgungs-, Signal-, Kontroll- und/oder Steuerleitungen (350,352) geführt sind, und an dessen Außenseite mindestens ein Regel-, Schalt- und Steuerorgan (310) angeordnet ist, **dadurch gekennzeichnet, daß** das Gehäuse (200) zur Haut hin von einer im wesentlichen planaren, auf die zu untersuchende Hautstelle ohne Einbringung einer Immersionsflüssigkeit aufbringbaren Platte (210) aus transparentem Kunststoff oder Glasmaterial begrenzt ist und die zwischen Beleuchtungseinrichtung (500) und Transparentplatte (210) und/oder zwischen Transparentplatte (210) und optischer Beobachtungseinrichtung (400) bzw. deren Vergrößerungsobjektiv anderseits angeordnete Lichtpolarisationseinrichtung (610,650) polarisationsform- und/oder polarisationsgradund/oder polarisationswinkel-variabel steuerbar und/oder abschaltbar bzw. aus einem jeweiligen Lichtstrahlengang mechanisch ausbringbar ausgebildet ist.

2. Einrichtung nach Anspruch 1, **dadurch gekennzeichnet, daß** sie ein an ihrer der Haut (150) zugewandten Seite mit einer austauschbar lösbaren Transparentplatte (210), begrenztes, tubusartiges Gehäuse (200) aufweist, in dessen Innerem, in an sich bekannter Weise achsparallel oder koaxial eine Kleinst-Videokamera (400) angeordnet ist, vor deren Objektiv (410) mindestens ein um die Gehäuse- bzw. Kameraachse drehwinkel-verstellbares Polarisationsfilter (600) angeordnet ist und die elektrischen Versorgungs-, Signal-, Kontrollund/oder -Steuerleitungen (350,352) für Beleuchtungseinrichtung (500), Aufnahmegerät (400) und externe Steuer-, Wiedergabe- und/oder Speichereinrichtung (700), welche von mindestens einem ergonomisch optimierten, Regel-, Schalt- und Steuerorgan (310), ausschalt- und steuerbar sind, in Flachbandkabeltechnik ausgeführt sind.

3. Einrichtung nach Anspruch 1 oder 2, **dadurch gekennzeichnet, daß** in einem peripheren Ringraum (240) zwischen Kameraobjektivgehäuse (411) und Wand des Gerätegehäuses (200) eine Mehrzahl von auf einem transparenten Kunststoffträgerelement (515) angeordneten Beleuchtungselementen (510), angeordnet ist, an deren Haupt-Lichtabstrahlungsseite ein mit einem unterseitig abgeschrägten, lichtstreuendoberflächenbehandelten Ringprisma aus transparentem Glas- oder Kunststoffmaterial, gebildetes Auflichtstrahl-Bündelungs- oder Diffusororgan (520) angeordnet ist.

4. Einrichtung nach einem der Ansprüche 1 bis 3, **dadurch gekennzeichnet, daß** das Gehäuse (200) mehrteilig mit einem die Transparent-Platte (210) und auch die Beleuchtungseinrichtung (500) tragenden, vorderen Gehäuseteil (201) und einem - im wesentlichen mit demselben fluchtend - im Abstand von diesem angeordneten, eine Videokamera (400) beherbergenden, hinteren Gehäuseteil (202) ausgebildet ist, wobei der Zwischenraum (212) zwischen vorderem und hinterem Gehäuseteil (201,202) vom Ringteil (622) des Polarisationsfilter-Halterungsorgans (620) durchsetzt ist und dieser Ringteil (622) seinerseits mit einem an den Außenseiten beider Gehäuseteile (201,202) gleitführungsgelagerten, dieselben achsial-unverschieblich haltenden, drehmanipulierbaren Überwurfzylinder (630), mit gerippter oder gerauhter Außenseite, verbunden ist.

5. Einrichtung nach Anspruch 4, **dadurch gekennzeichnet, daß** das Gehäuse (200) einen den vorderen (201) und den hinteren Gehäuseteil (202) umfassenden Gehäuse-Drehteil (221) mit dem an den genannten Gehäuseteilen (201,202) gleitdrehbar gelagertem Analysator-Halterungs- und Drehorgan (620) aufweist, welcher Gehäuse-Drehteil seinerseits, mittels Gewinde lösbar mit einem den im Winkel wegragenden Gehäusefortsatz (300) tragenden Gehäusebasisteil (203) verbunden ist.

6. Einrichtung nach einem der Ansprüche 1 bis 5, **dadurch gekennzeichnet, daß** das Analysator- Polarisationsfilter (600) an einem es umfassend haltenden, den Objektivteil (410) koaxial, etwa röhrenförmig umgebenden Vorsprungteil (621) eines den genannten Vorsprungteil und einen von diesem sich radial wegerstreckenden, an die Außenseite des Gehäuses (200) geführten Ringteil (622) aufweisenden Polarisationsfilter-Halterungs- und -Drehorgan (620) befestigt ist.

7. Einrichtung gemäß Anspruch 1, **dadurch gekennzeichnet, daß** sie ein einen Betrachtungskopf bildendes, verkürztes Gehäuse (200) mit an seiner Außenseite gleitdrehbar gelagertem, die Transparentplatte (210) und einen das Analysator-Polarisationsfilter (600) tragenden Halterungsteil (620) aufweisenden Überwurfzylinder (630), mit gerippter oder gerauhter Außenseite besitzt, und daß im Inneren des sich von dem Gehäuse (200) schräg oder im rechten Winkel radial wegerstreckenden Gehäusefortsatzes (300) peripheral eine mindestens ein Lichtemissionselement (510) umfassende Beleuchtungseinrichtung (500) mit in das Gehäuse ragendem, peripheralem Lichtleit- und Lichtemissionselement aus der Gruppe Lichtleit-Rohr (550), an seiner Innen- und Außenwand mit lichtreflektierendem Material beschichtetes Lichtleitrohr oder Faser-Lichtleitelement, weiters zentral ein von demselben umgebenes Bildaufnahmegerät (400) und mindestens ein Lichtumlenkelement (560), für das von einer zu untersuchenden Hautstelle (151) reflektierte Licht angeordnet sind.

8. Einrichtung nach einem der Ansprüche 1 bis 7, **dadurch gekennzeichnet, daß** die Beleuchtungseinrichtung (500) in einem mit dem Gehäusefortsatz (300), vorzugsweise mittels Gewinde, lösbar verbundenen, distalen Fortsatz-Endteil (325) angeordnet ist und eine Beleuchtungshalterung (515) aus Transparentmaterial mit radialen Ausnehmungen (516) für die Aufnahme einer Mehrzahl von austauschbaren Lichtemissionselementen mit Parabolreflektoren (517) umfaßt, welche Beleuchtungshalterung (515) im Gebrauchszustand mit ihren Endflächen (515') an die Endflächen des sich in das Gehäuse (200) erstreckenden Lichtleit- und Lichtemissionsorgans (550) satt anliegend ausgebildet ist.

9. Einrichtung nach einem der Ansprüche 5 bis 8, **dadurch gekennzeichnet, daß** die Beleuchtungseinrichtung (500) an der dem Objektiv (410) des optischen Aufnahmegerätes, abgewandten Seite, also hinter demselben angeordnet ist, oder aber daß sie überhaupt extern angeordnet ist und die Lichtzufuhr durch mindestens ein in die Einrichtung (100) geführtes Lichtleitkabel erfolgt.

10. Einrichtung nach einem der Ansprüche 5 bis 9, **dadurch gekennzeichnet, daß** die elektrische Versorgungs-, Signal-, Daten-, Kontroll- und Steuerleitungen (350-352) durch den Gehäusefortsatz-Endteil (325) geführt sind und am genannten Endteil (325) oder am Gehäusefortsatz (300) selbst die Schalt- und Regelorgane (310 bis 313) für Beleuchtung, Kamerafunktionen, eventuelle Polarisationsfunktionen und externen Computer (700), bevorzugt Trackball (311) und Mikroschalter (312, 313), angeordnet sind.

11. Einrichtung nach einem der Ansprüche 5 bis 10, **dadurch gekennzeichnet, daß** im Gehäusefortsatz-Endteil (325) zumindest Teile einer Steuerungselektronik für die Schaltorgane (310 bis 313) und zumindest eine mit einer entsprechenden Kontaktsteckeinheit (356) des Gehäusefortsatzes (300) leitverbindungs-kooperierende Leitungskontakt-Steckeinheit (357) angeordnet sind.

12. Einrichtung nach einem der Ansprüche 1 bis 11, **dadurch gekennzeichnet, daß** die elektrischen Versorgungsleitungen und -komponenten des Bildaufnahmegerätes (400) vom übrigen Gehäuse (200,300) und vom restlichen Versorgungs- und Leitungssystem (310,312,313) galvanisch getrennt ist.

13. Einrichtung nach Anspruch 1, **dadurch gekennzeichnet, daß** sie ein, von der auf die zu untersuchende Haut aufzulegenden Transparentplatte (210) abgeschlossenes, einen Betrachtungskopf bildendes, verkürztes Gehäuse (200) mit dem sich von diesem wegerstreckenden Gehäusefortsatz (300) aufweist, wobei im Gehäuse (200) - im geometrischen Durchdringungsraum desselben mit dem Gehäusefortsatz (300) und an die Transparentplatte (210) unmittelbar anschließend - ein partiell lichtreflektiveslichttransparentes Lichtleit- und -umlenkelement (560), wie Würfelprisma oder halbverspiegelte Transparentplatte, angeordnet ist, an welches sich in Fortsetzung der optischen Achse des Gehäuses die (Polarisator-)Polarisationseinrichtung (650) und eine lösbar ausgebildete, mit mindestens einem Lichtemissionselement (510) ausgestattete Beleuchtungseinrichtung (500) mit oder ohne Reflektor anschließt, und daß entlang der optischen Achse des Gehäusefortsatzes (300) vom Lichtleit- und -umlenkelement (560) weg die drehwinkelver- und -einstellbare Analysator-Polarisationseinrichtung (600) und ein optisches Aufnahmegerät (400) angeordnet ist.

14. Einrichtung nach Anspruch 13, **dadurch gekennzeichnet, daß** in ihrem Gehäusefortsatz (300) ein von einer, beispielsweise an anderer Stelle innerhalb des Gehäusefortsatzes untergebrachten Kameraelektronik getrennter, mittels Stellorgan (401) in Richtung der Gehäusefortsatz(300)-Achse verschieblich fokus-einstellbarer Videosensor (405), bevorzugt ein CCD-Chip, und ein Objektiv (410) angeordnet sind.

15. Einrichtung nach einem der Ansprüche 1 bis 14, **dadurch gekennzeichnet, daß** zur Reduktion der thermischen Belastung der Haut durch das bei einer Untersuchung auf dieselbe einstrahlendes Licht eine optoelektronische Schaltung mit sekundärem, schwachintensivem Schalt-Lichtemitter, vorzugsweise LED, und diesem zugeordnetem, auf von der Haut reflektiertes Emitter-Licht intensitätseinstellbar reagierendem Photosensor, vorgesehen ist, wobei bei Annäherung der Transparentplatte (210) an die Haut (150) bzw. bei Entfernung von derselben - jeweils abstandseinstellbar - das zur Untersuchung und Darstellung des jeweiligen Hautabschnittes einstrahlende Licht automatisch ab- bzw. einschaltbar ist.

16. Einrichtung nach einem der Ansprüche 1 bis 15, **dadurch gekennzeichnet, daß** sie zur Reduktion der thermischen Belastung der Haut eine Zeitschalteinrichtung aufweist, durch welche die Beleuchtungseinrichtung (500), bei - in der Zeitdauer einstellbarer und feststellbarer-Nichtaktivierung übriger, im Rahmen einer laufenden Untersuchung vom Arzt zu bedienender Schaltorgane (310 bis 313) od.dgl. der Untersuchtungseinrichtung (100) selbsttätig ausschaltbar ist.

17. Verfahren zur optischen Untersuchung von Humanhaut, insbesondere zur reproduzierbaren Erkennung und Untersuchung von pigmentalen Hautläsionen, bevorzugt unter Einsatz einer optischen Untersuchungs-Einrichtung gemäß einem der Ansprüche 1 bis 16, wobei eine zu untersuchende Stelle der Haut beleuchtet wird und das durch den von der Hautstelle reflektierten Lichtanteil generierte Bild, bevorzugt nach optischer Vergrößerung, direkt und/oder mittels Aufnahmegerät gespeichert wird und wobei durch drehwinkelver- und - einstellbare Polarisationsfilter, in den Lichtstrahlengängen Polarisation bzw. Polarisations-Analyse vorgenommen wird, **dadurch gekennzeichnet, daß** die zu untersuchende Stelle der Haut durch direktes Aufbringen einer, vorzugsweise planaren, Transparentplatte ohne Einbringung einer Immersionsflüssigkeit gespannt, geglättet und durch die Platte hindurch beleuchtet wird und daß - neben einer an sich bekannten Kombination einer Polarisation des auf die Hautstelle aufgestrahlten Lichtes mit einer Polarisationsanalyse des von der Hautstelle, insbesondere von der Oberhaut und der dermaepidermalen Junktionszone zwischen Ober- und Unterhaut wieder reflektierten Lichtes - gesondert davon eine Polarisation des aufgestrahlten, bevorzugt monochromatischen, Lichtes ohne Analyse des reflektierten Lichtes und/oder eine Polarisationsanalyse des von der Hautstelle reflektierten Lichtes ohne Polarisation des auf dieselbe aufgestrahlten Lichtes vorgenommen wird.

18. Verfahren nach Anspruch 17, **dadurch gekennzeichnet, daß** eine jeweils zu untersuchende Hautstelle in voneinander getrennten, bevorzugt sequentiellen, Stufen mit nicht polarisiertem Auflicht ohne Polarisations-Analysator im Strahlengang des von der Haut reflektierten Lichtes oder mit zueinander lichtschwingungsebenen-parallelem Polarisator und Analysator, weiters mit nicht polarisiertem Auflicht und zugeschaltetem Polarisations-Analysator, vorzugsweise vergleichend, untersucht und beobachtet wird und die dabei gewonnenen Abbildungen der Hautstelle aufgenommen und in einer Dokumentationseinrichtung gespeichert werden.

19. Verfahren nach Anspruch 17 oder 18, **dadurch gekennzeichnet, daß** auf eine jeweils zu untersuchende Hautstelle flächig-diffuses polarisiertes oder nicht polarisiertes Licht eingestrahlt wird.

20. Verfahren nach einem der Ansprüche 17 bis 19, **dadurch gekennzeichnet, daß** auf eine jeweils zu untersuchende Hautstelle elliptisch polarisiertes Licht eingestrahlt wird und/oder das reflektierte Licht durch einen elliptisch polarisierenden Analysator geführt wird.

21. Verfahren nach einem der Ansprüche 17 bis 20, **dadurch gekennzeichnet, daß** zur Reduktion der thermischen Belastung der Haut durch das bei einer Untersuchung auf dieselbe einstrahlende Licht mittels optoelektronischer Schaltung, vorzugsweise mittels sekundärem, schwachintensivem Schalt-Lichtemitter, z.B. LED, und diesem zugeordnetem, auf von der Haut reflektiertes Emitter-Licht intensitätseinstellbar reagierendem Photosensor, bei Annäherung der Transparentplatte an die Haut bzw. bei Entfernung von derselben jeweils abstandseinstellbar ein Ein- bzw. Ausschalten des zur Untersuchung und Darstellung des jeweiligen Hautabschnittes einstrahlenden Lichtes automatisch vorgenommen wird.

22. Verfahren nach einem der Ansprüche 17 bis 21 **dadurch gekennzeichnet, daß**, insbesondere zur Reduktion der thermischen Belastung der Haut mittels Zeitschaltung eine eingeschaltete Auflichtbeleuchtung, bei - in der Zeitdauer einstellbarer - Nichtaktivierung übriger, im Rahmen einer laufenden Untersuchung vom Arzt zu bedienender Schaltorgane der Untersuchtungseinrichtung selbsttätig ausgeschaltet wird.

23. Verfahren zur Auswertung der von einem der Untersuchungsverfahren nach einem der Ansprüche 17 bis 22 erhaltenen Daten bzw. Bilddaten, **dadurch gekennzeichnet, daß** dieselben, insbesondere zur Diagnose und Klassifikation von Hautläsionen, einer, bevorzugt computergestützten Hautstellen-Bild-Verarbeitung, -Analysen und -Datenreduktion sowie einer auf Daten aus einem mindestens einen Bildspeicher aufweisenden, neuronalen Netzwerk basierenden, dokumentationsspeicher-gestützten, vergleichende Bildinhalts-Erkennung und/oder -Addition und/oder -Subtraktion unterworfen wird.

24. Verfahren nach Anspruch 23, **dadurch gekennzeichnet, daß** auf Basis eines aktuellen Hautstellenbildes eine vergleichende Objekterkennung mittels multiphasischer Segmentierung und/oder lokaler Schwellwertbildung einschließlich Klassifikation mit folgenden wesentlichen Schritten vorgenommen wird:
- Trennung der Signale bzw. Daten der pigmentierten Läsion von jenen der dieselbe umgebenden Haut durch multiphasische Segmentierung und/oder lokale Schwellwertbildung, vorzugsweise unter Heranziehung eines, bevorzugt aus dem Rot/Grün-Kanal des Bildaufnahmegerätes, insbesondere Videokamera derivierten, Schwarzweißbildes
- Reduktion des Bildes auf n, vorzugsweise 10 Relativ-Grauwerte, wobei jeweils die Extremstufen n für gesunde Haut und 1 für die dunkelste Stelle der aktuell beobachteten Läsion vorgesehen sind, unter Bildung eines umrißgenauen Bildes der Läsion
- Maskierung der Läsion
- Bildung eines ersten Binärbildes der Läsion und Bestimmung von deren Parametern, insbesondere Fläche, Umfang, Symmetrie, fraktale Dimension oder Begrenzung des Randes und Aspect ratio
- Beurteilung des Randes der Läsion unter Bildung eines zweiten Binärbildes aus den Graustufen n - 1, n - 2 und (n - 1) + (n - 2) und dessen Analyse, insbesondere bezüglich der Relation der Flächen von erstem und zweitem Binärbild zueinander sowie Umfang und fraktale Dimension des zweiten Binärbildes
- Beurteilung der Pigmentierung durch sequentielle Vermessung von deren dunklen Anteilen
- Beurteilung der Farbe und Farbverteilung durch Transfer der Abbildung (Image) vom Rot-Grün-Bau-Farbraum in den Hue-Saturation-Intensity (Farbton-Sättigungslntensitäts)-Farbraum und Darstellung der Färbung und der Variabilität der Objekte durch ein per definitionem Schwarz-Weiß-Bild
- Auswertung der derart reduzierten Daten und Vergleich mit bekannten Daten unter Einsatz multivariater Verfahren, insbesondere eines neuronalen Netzwerks, in welches eine große Anzahl, insbesondere mindestens 100 Abbildungen und ihnen zugeordnete nach der vorliegenden Methode gewonnene Bildauswertungsdaten von Läsionen mit bekannten, eindeutigen und in ein Klassifizierungsschema gebrachten Diagnosen eingespeichert sind
- Einteilung aktuell zu analysierenden Abbildungen in eine der Kategorien eines Diagnoseschlüssels mit mehreren Stufen, wobei folgende Stufeneinteilung bevorzugt ist:
1: unverdächtige Läsion
2: verdächtige Läsion
3: wahrscheinlich maligne Läsion
und
- Bekanntgabe der jeweils gefundenen Kategorie, und bevorzugterweise zusätzlich von derselben entsprechenden Behandlungsvorschlägen.

## Claims

1. A device for optical examination of human skin and its pigmentations, with a cylindrical housing in which are arranged an incident-light illuminating device and an optical viewing device having an optical magnifying device, a polarising device with polariser (650) and/or analyser (600) being provided in the path of the beam of light radiated from the illuminating device (500) in the direction of an area of skin (151) currently to be examined, and additionally or alternatively in the path of the light-beam reflected from the skin (150), and a hollow housing continuation (300) designed as a handling grip extending from the housing (200), electrical supply lines, signal lines, control lines and/or pilot lines (350, 352) being guided through the said continuation and at least one regulating, switching and controlling member (310) being arranged on its outside, **characterised in that** the housing (200) is delimited with respect to the skin by a substantially planar plate (210) which may be applied to the skin area under investigation without introduction of an immersion fluid, the said plate being made of a transparent plastics or glass material, and **in that** the light-polarising device (610, 650) arranged between illuminating device (500) and transparent plate (210) and/or between transparent plate (210) and optical viewing device (400) or its magnifying lens on the other hand is designed so as to be variably controllable and/or disconnectable in respect of the form and degree of polarisation and the polarising angle, or so as to be mechanically removable out of a particular light-beam path,

2. A device according to Claim 1, **characterised in that** it has a tube-body shaped housing (200) delimited on its side facing the skin (150) by an exchangeably releasable transparent plate (210), a miniature video camera (400) being arranged inside the said housing parallel to the axis or coaxially, in a way that is known per se, in front of the lens (410) of which video camera at least one polarising filter (600) adjustable in its angle of rotation about the axis of the housing or of the camera is arranged, and the electrical supply lines, signal lines, control lines and/or pilot lines (350, 352) for illuminating device (500), recording instrument (400) and external controlling, reproduction and/or storage device (700), which are disconnectable and controllable by at least one ergonomically optimised regulating, switching and controlling member (310), are realised with the use of ribbon-cable technology.

3. A device according to Claim 1 or 2, **characterised in that** a plurality of illumination members (510) arranged on a transparent plastics-material carrier member (515) is arranged in a peripheral annular chamber (240) between the camera-lens housing (411) and the wall of the instrument housing (200), an incident light-concentrating or diffusing member (520), formed with an annular prism bevelled on the under side, surface-treated so as to scatter light and made of transparent glass material or plastics material, being arranged on the main light-radiating side of the said illumination members.

4. A device according to one of Claims 1 to 3, **characterised in that** the housing (200) is formed of many parts with an anterior housing part (201), bearing the transparent plate (210) as well as the illuminating device (500), and a posterior housing part (202) - arranged substantially in alignment with and at a distance from the said anterior housing part - which accommodates a video camera (400), the intermediate chamber (212) between anterior and posterior housing parts (201, 202) being penetrated by the annular part (622) of the polarising-filter retaining member (620) and this annular part (622) for its part being connected to a manually rotatable sleeve cylinder (630) with ribbed or roughened outside, the said cylinder being glidably mounted on the outsides of the two housing parts (201, 202) and holding the same axially undisplaceably.

5. A device according to Claim 4, **characterised in that** the housing (200) has a housing-rotating part (221) surrounding the anterior housing part (201) and the posterior (202) housing part, with the analyser-retaining and analyser-rotating member (620) glide-rotatably mounted on the aforementioned housing parts (201, 202), the said housing-rotating part for its part being - releasably by means of threads - connected to a housing base part (203) bearing the housing continuation (300) projecting at an angle.

6. A device according to one of Claims 1 to 5, **characterised in that** the analyser-polarising filter (600) is attached to projecting part (621) of a polarising-filter retaining and rotating member (620) which exhibits the aforementioned projecting part and an annular part (622) extending radially away from the said projecting part and guided on to the outside of the housing (200), the said projecting part retaining the said analyser-polarising filter by gripping around it and surrounding the lens part (410) coaxially, roughly in the form of a tube.

7. A device according to Claim 1, **characterised in that** it has a shortened housing (200) forming a viewing head, with a sleeve cylinder (630) glide-rotatably mounted on its outside, the said cylinder exhibiting the transparent plate (210) and a retaining part (620) which bears the analyser-polarising filter (600), and having a ribbed or roughened outside, and **in that** an illuminating device (500) comprising, at its periphery, at least one light-emitting member (510) and having, projecting into the housing, a peripheral light-conducting and light-emitting member from the light-conducting tube (550) group, a light-conducting tube or fibre light-conducting member coated on its internal and external wall with light-reflecting material, as well as centrally an image-recording instrument (400) enclosed by the said light-conducting tube or fibre light-conducting member and at least one light-deflecting member (560) are arranged - for the light reflected from an area of skin (151) to be examined - inside the housing continuation (300) extending radially away from the housing (200) diagonally or in a right angle.

8. A device according to one of Claims 1 to 7, **characterised in that** the illuminating device (500) is arranged in a distal continuation end part (325) releasably connected to the housing continuation (300), preferably by means of threads, and comprises an illumination mounting (515) made of a transparent material with radial recesses (516) for accommodating a plurality of exchangeable light-emitting members with parabolic reflectors (517), the said illuminating device (515) in the usable state being constructed with its end surfaces (515') fitting closely against the end surfaces of the light-conducting and light-emitting member (550) extending into the housing (200).

9. A device according to one of Claims 5 to 8, **characterised in that** the illuminating device (500) is arranged on the side facing away from the lens (410) of the optical recording instrument, i.e. behind the said instrument, or that it is actually arranged externally and the light enters through at least one light-conducting cable guided into the device (100).

10. A device according to one of Claims 5 to 9, **characterised in that** the electrical supply lines, signal lines, data lines, control lines and pilot lines (350 - 352) are guided through the housing-continuation end part (325) and the switching and regulating members (310 to 313) for illumination, camera functions, any polarisation functions, and external computer (700), preferably track ball (311) and microswitches (312, 313) are arranged on the aforementioned end part (325) or on the housing continuation (300) itself.

11. A device according to one of Claims 5 to 10, **characterised in that** at least parts of a control electronics system for the switching members (310 to 313) and at least one line-contact plug-in unit (357) co-operating to form a conducting connection with a corresponding contact plug-in unit (356) of the housing continuation (300) are arranged in the housing-continuation end part (325).

12. A device according to one of Claims 1 to 11, **characterised in that** the electrical supply lines and supply components of the image-recording instrument (400) are galvanically separated from the rest of the housing (200, 300) and from the rest of the supply-and-conduction system (310. 312, 313).

13. A device according to Claim 1, **characterised in that** it has a shortened housing (200) which is closed off from the transparent plate (210) to be laid on to the skin about to be examined and forms a viewing head, with the housing continuation (300) extending away from the said housing, a partially light-reflecting, light-transparent, light-conducting and light-deflecting member (560), such as a cubic prism or a semi-metallised transparent plate being arranged in the housing (200) - in the geometric penetration chamber of the same, with the housing continuation (300) and directly adjacent to the transparent plate (210), the (polariser-)polarising unit (650) and an illuminating device (500) of releasable construction, equipped with at least one light-emitting member (510) with or without reflector adjoining the said light-conducting and light-deflecting member in continuation of the optical axis of the housing, and **in that** the analyser-polarising unit (600) displaceable and adjustable about an angle of rotation, and an optical recording instrument (400) are arranged along the optical axis of the housing continuation (300) away from the light-conducting and light-deflecting member (560).

14. A device according to Claim 13, **characterised in that** a video sensor (405), preferably a CCD chip, which is separate from a camera electronics system accommodated, for example, in a different place within the housing continuation, and displaceably focus-adjustable by means of a control unit (401) in the direction of the axis of the housing continuation (300), and a lens (410), are arranged in the housing continuation (300) of the said device.

15. A device according to one of Claims 1 to 14, **characterised in that**, to reduce heat stressing of the skin due to the light radiated on to it during an examination, an optoelectronic switch with secondary, weak-intensive switching light-emitter, preferably an LED, and with a photosensor which reacts with adjustable intensity to emitter light reflected by the skin and is associated with the said light emitter is provided, the incident light for examining and displaying the particular section of skin being capable of being switched on or off automatically while moving the transparent plate (210) close to or away from the skin (150), in each case distance-adjustably.

16. A device according to one of Claims 1 to 15, **characterised in that**, to reduce heat stressing of the skin, it has a time-switch device, by means of which the illuminating device (500) can switch itself off automatically in the case of the time-adjustable and ascertainable non-activation of other switching members (310 to 313) or the like of the examining device (100), to be used by the doctor in the context of an on-going examination.

17. A method of optically examining human skin, in particular for the reproducible identification and examination of pigmented skin lesions, preferably with the use of an optical examination device according to one of Claims 1 to 16, in which an area of skin to be examined is illuminated and the image generated by the portion of light reflected by the skin is stored, preferably following optical magnification, directly and/or by means of a recording instrument, and in which polarisation or polarisation analysis is carried out in the paths of the light beams through polarising filters displaceable and adjustable about an angle of rotation, **characterised in that** area of skin to be examined is tensioned by direct application of a - preferably planar - transparent plate without introducing an immersion fluid, smoothed, and light is passed through the plate, and **in that** - in addition to and separately from a combination - known per se - of polarisation of the light irradiated on to the skin area with polarisation analysis of the light re-reflected by the skin area, in particular by the epidermis and the epidermal junction zone between the epidermis and the subcutaneous cell tissue, polarisation of the incidental, preferably monochromatic light is performed without analysis of the reflected light, and/or polarisation analysis of the light reflected by the skin area is performed without polarisation of the light radiated on to the said skin.

18. A method according to Claim 17, **characterised in that** a particular area of skin to be examined is examined and viewed in separate, preferably sequential stages with non-polarised incident light without a polarisation analyser in the path of the light reflected by the skin, or with polariser and analyser parallel to one another in respect of the light-oscillation plane, furthermore with non-polarised incident light and connected polarisation analyser, preferably comparatively, and the images of the skin area thereby obtained are recorded and stored in an indexing device.

19. A method according to Claim 17 or 18, **characterised in that** extensively diffuse polarised or non-polarised light is radiated on to a particular area of skin to be examined.

20. A method according to one of Claims 17 to 19, **characterised in that** a particular area of skin to be examined is irradiated with elliptically polarised light and/or the reflected light is guided through an elliptically polarising analyser.

21. A method according to one of Claims 17 to 20, **characterised in that**, to reduce heat stressing of the skin due to the light radiated on to the said skin during an examination by means of optoelectronic switching, preferably by means of a secondary, switchable light emitter of weak intensity, e.g. an LED, and a photosensor intensity-adjustably reactive to the emitter light and associated with the said light emitter, switching on or off of the incident light for examining and displaying the particular skin section is performed automatically while moving the transparent plate close to or away from the skin, in each case distance-adjustably.

22. A method according to one of Claims 17 to 21, **characterised in that** in particular to reduce heat stressing of the skin by means of a time switch, an incident-light illuminating device connected in the circuit switches itself off during non-activation - adjustable in duration - of other switching members of the examining device that are to be operated in the context of an examination currently being performed by the doctor.

23. A method of evaluating the data of image arrays obtained by one of the methods of examination according to one of Claims 17 to 22, **characterised in that**. in particular for the diagnosis and classification of skin lesions, these data or arrays being subjected to a preferably computer-supported skin-area image processing, image analysis and image data reduction, and documentation store-supported comparative image-content recognition and/or -addition or -subtraction based on data from a neural network having at least one image-storing device.

24. A method according to Claim 23, **characterised in that**, on the basis of a current image of the skin area, comparative object recognition is performed by means of multiphase segmentation and/or local threshold-value formation including classification, with the following important steps:
- Separation of the signals or data of the pigmented lesion from those of the skin area surrounding it by multiphase segmentation and/or local threshold-value formation, preferably using a black-and-white image preferably derived from the red-green channel of the image-recording device, in particular a video camera;
- Reduction of the image to n, preferably 10 relative grey-scale values, the extreme values provided in each case being n for healthy skin and 1 for the darkest area of the lesion currently being viewed, with formation of an image of the lesion with a precise outline;
- Masking of the lesion;
- Formation of a first binary image of the lesion and determination of its parameters, in particular area, circumference, symmetry, fractal dimension or margin definition and aspect ratio;
- Evaluation of the lesion margin with formation of a second binary image from the grey-scale levels n - 1, n - 2 and (n - 1) + (n - 2) and its analysis, in particular with reference to the relationship between the areas of the first and second binary image to one another and the circumference and fractal dimension of the second binary image;
- Evaluation of the pigmentation by sequential measurement of their dark portions;
- Evaluation of the colour and colour distribution by transferring the image from the red-green-blue colour space to the hue-saturation-intensity colour space and displaying the colour and variability of the objects via a by-definition black-and-white image;
- Evaluation of the thus reduced data and comparison with known data with the use of multivariate methods, in particular of a neuronal network, in which a large number, in particular at least 100 images and image evaluation data - obtained by the present method and associated therewith - of lesions with known, unambiguous and classified diagnoses are stored;
- Division of images currently to be analysed into one of the categories of a diagnostic key with a plurality of steps, the following grading being preferred:
1. non-suspicious lesion
2. suspicious lesion
3. probably malignant lesion
and
- Notification of the category found in each case, and preferably also corresponding suggested treatments.

## Revendications

1. Dispositif pour l'examen optique de la peau humaine et de sa pigmentation comprenant un boîtier en forme d'un cylindre, dans lequel un dispositif d'observation optique, incluant un dispositif d'agrandissement optique, et un dispositif d'éclairage incident sont aménagés, un dispositif de polarisation ayant un polariseur (650) et/ou un analyseur (600) étant disposé dans la marche des rayons de la lumière irradiée du dispositif d'éclairage (500) dirigée à un endroit de peau (151) actuellement à examiner et additionellement ou alternativement dans la marche des rayons de la lumière réfléchie de la peau (150), et un prolongement du boîtier (300) creux formé comme poignée de manipulation saillant du boîtier (200), par lequel des lignes d'alimentation, de signaux, de contrôle et/ou de commande (350, 352) sont amenées et à l'extérieur duquel au moins un organe de réglage, de coupure et de commande (310) est disposé, **caractérisé en ce que** le boîtier (200) est délimité vers la peau par une plaque (210) d'une matière artificielle transparente ou d'un matériau vitreux substantiellement planaire et applicable à l'endroit de peau à examiner sans introduction d'un liquide d'immersion, et que le dispositif de polarisation de lumière (610, 650) disposé entre le dispositif d'éclairage (500) et la plaque transparente (210) et/ou entre la plaque transparente (210) et le dispositif d'observation optique (400) ou l'objectif d'agrandissement duquel d'autre part peut être commandé à l'égard de la forme de polarisation et/ou du degré de polarisation et/ou en variant l'angle de polarisation et/ou mis hors circuit et/ou ôté mécaniquement hors de la marche des rayons de lumière respective.

2. Dispositif selon la revendication 1, **caractérisé en ce qu'**il a, à son côté tourné vers la peau (150), un boîtier tubulaire (200) délimité par une plaque transparente (210) échangeable et détachable, à l'intérieur duquel une mini-caméra photo-vidéo est disposée parallèlement à l'axe ou coaxialement d'une manière connue en soi, avant de l'objectif (410) de laquelle au moins un polaroïd (600) à l'angle d'orientation variable autour de l'axe du boîtier ou de la caméra est disposé, et que les lignes d'alimentation, de signaux, de contrôle et/ou de commande (350, 352) pour le dispositif d'éclairage (500), l'appareil d'enregistrement (400) et un dispositif externe de commande, de reproduction et/ou de mémorisation (700), qui peuvent être mis hors de circuit et commandés par au moins un organe de réglage, de coupure et de commande (310), sont formés en technique de câble à ruban.

3. Dispositif selon la revendication 1 ou 2, **caractérisé en ce que** plusieurs éléments d'éclairage (510), disposés sur un élément de support de matière artificielle transparente (515), sont placés dans un espace annulaire périphérique (240) entre le boîtier d'objective de caméra (411) et un paroi du boîtier de l'appareil (200), au côté principal de rayonnement de lumière desquels un organe de concentration ou diffusant des rayons d'éclairage incidents (520) est disposé et formé avec un prisme annulaire d'un matériau vitreux ou d'une matière artificielle transparente, dont le côté bas est incliné, dont la surface est traitée de manière à être dispersive.

4. Dispositif selon une quelconque des revendications 1 à 3, **caractérisé en ce que** le boîtier (200) est composé et comprend une partie de boîtier (201) avant, qui porte la plaque transparente (210) et aussi le dispositif d'éclairage (500), et une partie de boîtier (202) arrière, qui est substantiellement affleurant avec celui-ci, est espacé de celui-ci et loge une caméra photo-vidéo (400), l'espace intermédiaire (212) entre la partie avant et arrière (201, 202) étant traversé par la partie annulaire (622) de l'organe d'attache du polaroïd (620) et ladite partie annulaire (622) étant liée de sa part à un cylindre d'accouplement (630) ayant une surface extérieure cannelée ou rugueuse à manipuler en rotation, qui est supporté de manière coulissante aux faces extérieures des deux parties de boîtier (201, 202) en les tenant axialement fixées.

5. Dispositif selon la revendication 4, **caractérisé en ce que** le boîtier (200) comprend une partie tournante de boîtier (221) incluant la partie de boîtier avant (201) et la partie de boîtier arrière (202) et ayant l'organe d'attache d'analyseur rotatif (620) logé de manière coulissante et rotative, ladite partie tournante de boîtier de sa part étant reliée à une partie de base du boîtier (203), qui porte le prolongement du boîtier (300) saillant sous un angle, de manière détachable par moyens d'un filetage.

6. Dispositif selon une quelconque des revendications 1 à 5, **caractérisé en ce que** le polaroïd analyseur (600) est fixé à une partie de prolongement (621) d'un organe porte polaroïd rotatif (620), qui le porte en le tenant embrassé et entourant la partie d'objectif (410) coaxialement et d'une manière approximativement tubulaire, ledit organe comprenant ladite partie de prolongement et une partie annulaire (622) saillant radialement de la dernière et s'étendant à la face extérieure du boîtier (200).

7. Dispositif selon la revendication 1, **caractérisé en ce qu'**il a un boîtier (200) raccourci formant une tête d'observation et comprenant à sa surface extérieure le cylindre d'accouplement (630) ayant une surface extérieure cannelée ou rugueuse, étant logé de manière coulissante et rotative, qui comprend la plaque transparente (210) et une partie d'attache (620) portant le polaroïd analyseur (600), et qu'un dispositif d'éclairage (500) est disposé péripheralement à l'intérieur du prolongement de boîtier (300), saillant radialement sous un angle incliné ou droit du boîtier (200), et est prévu d'au moins un élément d'émission de lumière (510) périphéral avec un élément de guidage et d'émission de lumière du groupe comprenant un tube de guidage de lumière (550), tube de guidage de lumière, dont les surfaces interne et externe sont enduites d'un matériau réfléchissant la lumière, ou un élément de guidage à fibres optiques, qui s'étend dans le boîtier, et que, de plus, un appareil de prise de vue entouré par celui-ci et au moins un élément déviateur (560) de la lumière réfléchis par un endroit de peau (151) à examiner sont disposé centralement.

8. Dispositif selon une quelconque des revendications 1 à 7, **caractérisé en ce que** le dispositif d'éclairage (500) est disposé dans une partie finale (325) du prolongement distale, reliée de manière détachable au prolongement du boîtier (300), préférablement par un filetage, et comprend un porte-éclairage (515) d'un matériau transparent, qui a des vides radiales (516) pour recevoir plusieurs éléments d'émission de lumière remplaçables avec des réflecteurs paraboliques (517), les surfaces terminaux (515') dudit porte-éclairage (515) étant formé à joindre à plat point aux surfaces terminaux de l'élément de guidage et d'émission de lumière (550), qui s'étend dans le boîtier (200).

9. Dispositif selon une quelconque des revendications 5 à 8, **caractérisé en ce que** le dispositif d'éclairage (500) est disposé au côté détourné de l'objectif (410) de l'appareil optique de prise de vue, c'est à dire derrière de celui-ci, ou bien qu'il est disposé d'une façon générale externement, l'alimentation de lumière ayant lieu par au moins un câble optique amené dans le dispositif (100).

10. Dispositif selon une quelconque des revendications 5 à 9, **caractérisé en ce que** les lignes d'alimentation, de signaux, de dates, de contrôle et de commande (350-352) sont amenées à travers de la partie finale du prolongement de boîtier (325), et que les organes de coupure et de réglage (310 à 313) pour l'éclairage, les fonctions de la caméra, des fonctions éventuelles de polarisation et un ordinateur externe (700), préférablement un track ball (311) et un micro-interrupteur (312, 313), sont disposés à ladite partie finale (325) ou au prolongement de boîtier (300) lui-même.

11. Dispositif selon une quelconque des revendications 5 à 10, **caractérisé en ce qu'**au moins des parts d'une électronique de commande pour les organes de coupure (310 à 313) et au moins une unité connecteuse de ligne (357) coopérant avec une unité connecteuse (356) du prolongement de boîtier (300) correspondante pour la connexion des lignes sont disposées dans la partie finale (325) du prolongement de boîtier.

12. Dispositif selon une quelconque des revendications 1 à 11, **caractérisé en ce que** les lignes et composants électriques d'alimentation de l'appareil de prise de vue (400) sont galvaniquement séparés du reste du boîtier (200, 300) et du reste du système d'alimentation et des lignes (310, 312, 313).

13. Dispositif selon la revendication 1, **caractérisé en ce qu'**il a un boîtier (200) raccourci formant une tête d'observation et fermé par la plaque transparente (210) à être appliquée à la peau à examiner, qui comprend le prolongement de boîtier (300) saillant de celui-ci, dans ledit boîtier (200) un élément de guidage de lumière et déviateur (560), comme un prisme cubique ou une plaque transparente semi-réfléchissante, partiellement réfléchissant la lumière et partiellement transparent à la lumière étant disposé dans l'espace géométrique de pénétration duquel avec le prolongement de boîtier (300) et immédiatement adjoint à la plaque transparente (210), auquel le dispositif de polarisation (polariseur) (650) et un dispositif d'éclairage (500) ayant un réflecteur ou non, étant formé d'une manière détachable et équipé d'au moins d'un élément d'émission de lumière (510) se joignent, et **en ce qu'**en partant de l'élément de guidage de lumière et déviateur (560), le dispositif de polarisation analyseur (600) à l'angle d'orientation variable et adjustable et un appareil de prise de vue (400) sont disposés le long de l'axe optique du prolongement de boîtier (300).

14. Dispositif selon la revendication 13, **caractérisé en ce que** dans son prolongement de boîtier (300) un détecteur vidéo (405), préférablement un chip CCD, séparé de l'électronique de caméra, qui est par exemple logée à une autre place à l'intérieur du prolongement de boîtier, ledit détecteur étant déplaçable et adjustable par rapport au point focale en direction de l'axe du prolongement de boîtier (300) au moyen d'un organe de réglage, ainsi qu'un objectif (410) sont disposés.

15. Dispositif selon une quelconque des revendications 1 à 14, **caractérisé en ce qu'**un circuit optoélectronique avec un émetteur de lumière commutable secondaire d'une intensité faible, préférablement une LED, pour réduire la charge thermique de la peau par la lumière incidente à elle pendant l'examen, et un photo-détecteur associé, réactif à la lumière de l'émetteur réfléchie par la peau d'une manière à être adjustable par rapport à l'intensité sont prévus, la lumière incidente pour l'examen et représentation du segment respectif de la peau étant mis en ou hors circuit automatiquement lors de l'approche de la plaque transparente (210) à la peau (150) et lors de l'éloignement de celle-ci, respectivement, la distance respective étant adjustable.

16. Dispositif selon une quelconque des revendications 1 à 15, **caractérisé en ce qu'**elle comprend un interrupteur à minuterie pour réduire la charge thermique de la peau, par lequel le dispositif d'éclairage (500) peut être interrompu automatiquement, tandis que des autres organes de commutation (310 à 313) ou pareil à contrôler par le médecin dans le cadre d'un examen courant ne sont pas activés de manière déterminable pendant une durée adjustable.

17. Procédé pour l'examen optique de la peau, particulièrement pour la reconnaissance reproductible et l'examen des lésions de peau pigmentales, préférablement en utilisant un dispositif d'examen optique selon une quelconque des revendications 1 à 16, dans lequel un endroit de la peau est éclairé et l'image générée par la part de lumière réfléchie par l'endroit de peau est enregistrée directement et/ou au moyen d'un appareil de prise de vue, préférablement après un agrandissement optique, et dans lequel une polarisation et/ou une analyse de polarisation est effectuée par des polaroïds d'une orientation variable et adjustable, **caractérisé en ce que** par application directe d'une plaque transparente, préférablement planaire, sans introduction d'un liquide d'immersion, l'endroit de la peau à examiner est tendu, plané et éclairé à travers la plaque, et qu'outre une combinaison connue en soi d'une polarisation de la lumière irradiée à l'endroit de peau avec une analyse de polarisation de la lumière réfléchie par l'endroit de peau, particulièrement de l'épiderme et la zone de jonction derma-épidermale entre l'épiderme et la membrane adipeuse, on effectue séparément une polarisation de la lumière irradiée, préférablement monochromatique, sans une analyse de la lumière réfléchie et/ou une analyse de polarisation de la lumière réfléchie par l'endroit de peau sans un polarisation de la lumière irradiée à celui-ci.

18. Procédé selon la revendication 17, **caractérisé en ce qu'**un endroit de peau respectif à examiner est examiné et observé par étapes séparées, préférablement séquentielles, avec une lumière incidente, non-polarisée sans un analyseur de polarisation dans la marche des rayons de la lumière réfléchie de la peau ou avec un polariseur et un analyseur parallèles au plan de polarisation, et de plus avec la lumière incidente non-polarisée et un analyseur de polarisation monté est examiné et observé, préférablement en les comparant, et que les images de l'endroit de peau gagnées ainsi sont enregistrées et sont mémorisées dans un dispositif de documentation.

19. Procédé selon la revendication 17 ou 18, **caractérisé en ce que** l'on irradie de la lumière diffuse à deux dimensions, polarisée ou non à un endroit de peau à examiner respectif.

20. Procédé selon une quelconque des revendications 17 à 19, **caractérisé en ce que** l'on irradie de la lumière elliptiquement polarisée et/ou la lumière réfléchie est guidée à travers un analyseur polarisant elliptiquement.

21. Procédé selon une quelconque des revendications 17 à 20, **caractérisé en ce que** l'on effectue une mise en ou hors circuit automatique de la lumière incidente pour l'examen et la représentation de l'endroit de peau respectif lors de l'approche de la plaque transparente à la peau et lors de l'éloignement de celle-ci, respectivement, la distance respective étant adjustable, pour réduire la charge thermique de la peau par la lumière y incidente lors de l'examen au moyen d'un circuit optoélectronique, préférablement par un émetteur de lumière secondaire commutable d'une intensité faible, p.e. une LED, et un photodétecteur associé, réactif à la lumière de l'émetteur réfléchie par la peau d'une manière à être adjustable par rapport à l'intensité.

22. Procédé selon une quelconque des revendications 17 à 21, **caractérisé en ce qu'**un éclairage incident mis en circuit est interrompu automatiquement au moyen d'un interrupteur à minuterie, particulièrement pour réduire la charge thermique de la peau, tandis que des autres organes de commutation (310 à 313) du dispositif d'examen à contrôler par le médecin dans le cadre d'un examen courant ne sont pas activés de manière déterminable pendant une durée adjustable.

23. Procédé pour évaluer les données ou données-image obtenues par un procédé d'examen selon une quelconque des revendications 17 à 22, **caractérisé en ce que** ceux-ci sont soumis à un traitement d'images d'endroits de peau, d'une analyse d'images et d'une réduction des données, préférablement assisté par ordinateur, ainsi que d'une analyse d'image comparative et/ou addition et/ou soustraction basée sur des données d'un réseau neuronal comprenant au moins un mémoire image, assistée par un mémoire de documentation.

24. Procédé selon la revendication 23, **caractérisé en ce qu'**une reconnaissance des objets comparative à la base d'une image actuelle d'un endroit de peau est effectuée par segmentation multi-phasée et/ou une formation de valeurs seuil locaux incluant une classification avec les étapes essentielles suivantes:
- séparation des signaux ou données de la lésion pigmentée de ceux de la peau l'entourant par segmentation multi-phasée et/ou formation de valeurs seuil locaux, préférablement en utilisant une image en noir et blanc, de préférence dérivée d'un canal rouge et vert de l'appareille de prise de vue, particulièrement une caméra photo-vidéo,
- réduction de l'image à n, préférablement 10, valeurs de gris relatifs, les l'échelonnements extrêmes étant prévus n pour une peau saine et 1 pour le lieu plus obscur de la lésion actuellement observée, en formant une image à contours précises de la lésion
- masquage de la lésion
- formation d'une première image binaire de la lésion et détermination de ses paramètres, particulièrement de la superficie, le périmètre, la symétrie, la dimension fractale ou la délimitation des confins et l'*aspect ratio*
- jugement des confins de la lésion en formant une deuxième image binaire des valeurs de gris n - 1, n - 2 et (n - 1) + (n - 2) et analyse de celle-ci, particulièrement par rapport à la relation mutuelle des superficies de la première image et la deuxième, ainsi que le périmètre et la dimension fractale de la deuxième image binaire
- jugement de la pigmentation par mesurage séquentiel des parts obscures
- jugement de la couleur et distribution des couleurs par transfert de l'image de l'espace de couleurs rouge-vert-bleu dans l'espace de couleurs *hue-saturation-intensity* (nuance de couleur-saturation-intensité) et représentation de la coloration et de la variabilité des objets par une image en noir et blanc *per definitionem*
- évaluation des données ainsi réduites et comparaison avec des données connues en utilisant un procédé multivariable, particulièrement d'un réseau neuronal, dans lequel un grand nombre, particulièrement au moins 100 images et des données d'évaluation d'image associées des lésions, gagnées selon la méthode présente avec des diagnoses connues, définies et organisée dans un schéma de classification est mémorisé
- triage des images actuellement à analyser dans une des catégories d'un clé de diagnose ayant plusieurs dégréés, la classification suivante étant préférée:
1: lésion non suspecte
2: lésion suspecte
3: lésion probablement maligne
et
- publication de la catégorie trouvée respective et, de préférence, additionellement des propositions de traitement correspondantes à elle.
